# EUROPÄISCHE PATENTSCHRIFT

(11) **EP 3 897 343 B1**
(45) Veröffentlichungstag und Bekanntmachung des Hinweises auf die Patenterteilung: **30.03.2022**
(21) Anmeldenummer: 20723509.4
(22) Anmeldetag: 11.03.2020
(51) Int. Cl.: A61B 1/00, A61B 1/015, A61B 1/018

(54) **ENDOSKOP MIT SCHNELLWECHSELSCHLÄUCHEN**
ENDOSCOPE COMPRISING QUICK-CHANGE TUBES
ENDOSCOPE POURVU DE TUBES À CHANGEMENT RAPIDE

(30) Priorität: 04.03.2019 DE 102019105438
(43) Veröffentlichungstag der Anmeldung: 27.10.2021
(73) Patentinhaber: es endomed solutions GmbH, 84051 Essenbach (DE)
(72) Erfinder: KRESS, Jürgen, 84051 Essenbach (DE)
(74) Vertreter: Pöhner, Wilfried Anton
(86) Internationale Anmeldenummer: PCT/IB2020/000131
(87) Internationale Veröffentlichungsnummer: WO 2020/178634

(56) Entgegenhaltungen:
- EP-A1- 1 284 120
- DE-A1-102012 105 370
- US-A- 4 947 827

## Beschreibung

Diese Erfindung bezieht sich auf Endoskope zur minimalinvasiven Untersuchung und chirurgischen Behandlung von Patienten, genauer auf Endoskope mit Kanälen zum Führen eines chirurgischen Instrumentes oder zum Leiten von Wasser oder Luft während eines medizinischen Eingriffs, wobei die Endoskope eine schnelle Sterilisierung und Wiederverwendung erlauben. Weiterhin stellt die Erfindung Verfahren vor, derartige Endoskope zur (Wieder-)Verwendung vor und nach einer Untersuchung oder Operation bereit zu machen.

Endoskope sind Instrumente zur Durchführung von minimalinvasiven Diagnosen und operativen Eingriffen in Kavitäten und Hohlorganen von Patienten, wobei im Vergleich zu offenen Verfahren nur ein Minimum an operativem Trauma und Stress verursacht wird. Hierfür haben Endoskope einen Haltegriff, welcher an einem proximalen Ende eines langen, flexiblen, schlauchartigen Schaftes zur Einführung in eine Körperöffnung oder einen kleinen Einschnitt in der Haut des Patienten befestigt ist. Um dieses Einführen zu erleichtern und Gewebeschäden und Schmerzen für den Patienten so klein wie möglich zu halten, hat der Schaft eine glatte Außenhaut und ist so gestaltet, dass er einen möglichst kleinen Querschnitt bzw. Umfang aufweist. In seinem Inneren verlaufen optische Fasern bis zu seinem distalen Ende, um zum einen Licht an die Operationsstelle zu bringen und zum anderen die Operationsstelle auch optisch beobachten zu können und hierdurch eine visuelle Diagnose und eine visuelle Überwachung während chirurgischer Eingriffe zu ermöglichen. Als Alternative zu optischen Fasern kann eine Überwachung und Beobachtung auch mittels eines im distalen Ende des Schaftes integriertenein Kamerachips und eine Beleuchtung mittels einer Miniaturlichtquelle (wie beispielsweise eine LED) erfolgen, wobei diese jeweils über elektrische Kabel, die im Inneren des Schaftes verlaufen, angesteuert werden.

Während der Verwendung eines Endoskops wird die Endoskopspitze, also das distale Ende des Schaftes, zur gewünschten Stelle innerhalb des Patienten geleitet, indem im Bereich des distalen Endes der Schaftes während des Einschiebens in den Patienten in die entsprechende Richtung gebogen wird. Diese Biegung der Endoskopspitze, also des distalen Endes des Schaftes, kann bei Endoskopen durch mechanische Kabel oder Drähte (Bowdenzüge), welche im Inneren des Schaftes verlaufen, kontrolliert werden, wobei diese Kabel mit entsprechenden Steuermitteln, beispielsweise ergonomisch an eine Seite des Griffes platzierten Kontrollrädern, verbunden sind, und so die intuitive und einfache Verwendung des Endoskops erleichtern. Es sind auch hydraulisch bewegte Endoskope bekannt, bei denen zur Bewegung des distalen Endes ein Hydrauliksystem mit im Schaft integrierten Hydraulikkanälen vorhanden ist, welches mittels gut erreichbar am Griff angebrachte Schalter, beispielsweise Wippschalter, kontrolliert wird.

Um zusätzlich zu visuellen Untersuchungen auch chirurgische Eingriffe, wie beispielsweise Biopsien, zu ermöglichen, haben Schäfte bekannter Endoskope in ihrem Inneren einen axial verlaufenden Instrumenten- bzw. Instrumentierkanal, auch Biopsiekanal genannt, zum Führen eines drahtkontrollierten chirurgischen Instruments wie beispielsweise einer Zange, einer chirurgischen Schere, oder auch einer Schlinge. Dieser Kanal kann auch anderweitig Verwendung finden, beispielsweise um Wasser oder Luft zur Operationsstelle zu führen oder um, etwa zur Absaugung von Flüssigkeiten, einen Unterdruck an der Operationsstelle bereitzustellen. Da es jedoch oft wünschenswert ist, all diese Funktionen gleichzeitig bereitstellen zu können, um manche Eingriffe zu erleichtern oder überhaupt erst zu ermöglichen, können auch weitere Kanäle vorhanden sein, beispielsweise ein separater Kanal für jede der obigen Funktionen, oder zumindest separate Kanäle für die (Druck-)Luftinsufflation und die Wasserspülung. Diese Kanäle werden dann nach ihrer Funktion in Arbeitskanäle zum Führen von Instrumenten und/oder zur Absaugung von Flüssigkeiten und Versorgungskanäle unterschieden.

Dass durch den Wasserkanal geleitete Wasser kann zur Spülung und Reinigung der Operationsstelle oder der Instrumente oder der optischen Vorrichtung des Endoskops verwendet werden. Der (Druck-)Luftkanal oder ein kombinierter, Luft-/Unterdruckkanals können dazu eingesetzt werden, vorrübergehend Hohlräume von Organen um die Operationsstelle aufzublasen oder die Luft wieder abzusaugen, die Operationsstelle oder die verwendeten Instrumente zu trocknen oder um Ballons aufzublasen und dadurch Gefäße oder Hohlräume für längere Zeiten zu erweitern. Zur Absaugung von Flüssigkeiten wird jedoch üblicherweise der größere Instrumentierkanal eingesetzt.

Da ohne weitere Maßnahmen zumindest der Schaft des Endoskops in Kontakt mit den Körperflüssigkeiten des Patienten kommt, ist eine gründliche Reinigung oder eine Sterilisation zumindest dieses Schaftes, idealerweise aber des ganzen Endoskops, eine absolute Notwendigkeit. Jedoch ist solch eine Reinigung und Sterilisation bei der hier geforderten Gründlichkeit sehr arbeitsintensiv und zeitraubend. Dies gilt insbesondere für Endoskope mit Kanälen im Inneren des Schaftes, da diese unter Anschluss an eine Pumpe gründlich eine gewisse Zeit mit Desinfektionsflüssigkeit ausgespült werden müssen. Während der Sterilisation des Endoskops ist dieses nicht verfügbar. Von einem ökonomischen Standpunkt ist dies sehr nachteilig, da Endoskope üblicherweise kostspielige medizinische Geräte sind, und daher, um sie schnell zu automatisieren, der Wunsch besteht, sie so oft wie möglich einzusetzen.

Um die zur Wiederverwendung eines Endoskops benötigte Zeit zu reduzieren sind Schutzüberzüge bekannt, welche zumindest einen Teil oder auch den ganzen Schaft bedecken und auch weiter reichen können, um einen Teil des Griffs einzuschließen. Die Dokumente US 5,217,001 und WO 2004/060149 A1 offenbaren beispielsweise Schutzhüllen umfassend eine zumindest teilweise transparente Endkappe, welche über das distale Ende des Schaftes gesteckt wird und an der eine flexible, Donutförmig aufgerollte Hülle befestigt ist, welche entlang des Schaftes abgerollt werden kann, um diesen und möglicherweise auch den Griff des Endoskops teilweise oder ganz zu umschließen.

Eine ähnliche Endoskophülle ist auch in der DE10 2018 110 228 vorgeschlagen, mit dem Unterschied, dass dort eine zweischichtige Hülle verwendet wird, bei der eine zusätzliche äußere Schicht, bevor das Endoskop in den Patienten eingeführt wird, über das distale Ende des Endoskops hinausreicht und flüssigkeitsdicht an eine Anschlussvorrichtung, wie beispielsweise ein Mundstück für die Gastroskopie oder spezielle Hosen für eine Koloskopie, anschließbar ist, wodurch eine Kontamination der Außenseite bzw. des Endoskopverwenders wirksam verhindert werden kann.

Diese Schutzhüllen für Endoskope umfassen an die Endkappe befestigte hohle Schläuche, welche Arbeitskanäle, wie beispielsweise einen Instrumentierkanal, der auch als kombinierter Absaugkanal verwendbar ist, sowie Versorgungskanäle, wie etwa einen Wasser- oder einen (Druck-)Luftkanal der oben beschriebenen Art bereitstellen. Entweder, wie im Fall der DE10 2018 110 228, sollen diese als Innenauskleidung in einen bereits vorhandenen Kanal des Schaftes eingeführt werden, wobei sie als Barriere zwischen dem im Kanal geführten chirurgischen Instrument, Wasser, der Luft oder, insbesondere, eingesaugten Körperflüssigkeiten und biologischem Material und dem Endoskop dienen, oder die Schläuche sollen an der Außenseite des Schaftes entlang verlaufend verlegt werden, wie beispielsweise in der WO 2004/060149 A1 vorgeschlagen.

Im ersten Fall ist ein Nachteil, dass das Einschieben der relativ elastischen und weichen Schläuche durch den schmalen Kanal im Schaft ein sehr schwieriger und potentiell zeitraubender Schritt in der Vorbereitung des Endoskops darstellt. Im zweiten Fall, kommt es zu einer Erhöhung des effektiven Querschnitts des Schaftes, was die Gefahr einer Vergrößerung des Schmerzes und der Gewebeschäden beim Patienten mit sich bringt und somit das Endoskop für seinen angedachten Einsatzzweck als minimalinvasives Instrument weniger geeignet macht.

Weiterhin haben in den Schaft integrierte Biopsiekanäle den Vorteil, dass sie bei Verbiegen des Schaftes nur eine kleine Längenänderung erfahren und so die unbeabsichtigt auf ein im Biopsiekanal eingeführtes Instrument ausgeübten Kräfte ebenfalls nur klein sind. Falls der Biopsiekanal durch einen auf der Außenseite des Schaftes entlang geführten Schlauches bereitgestellt wird, ist dies nicht länger der Fall, was eine Einstellung und gezielte Kontrolle von chirurgischen Instrumenten während einer Operation erschwert.

Zwar schlägt die WO 2004/060149 A1 zur Verringerung des Problems der Querschnittsvergrößerung durch außen am Schaft anliegender Arbeitskanäle vor, in der Außenseite des Schaftes Kerben vorzusehen, jedoch werden in diesem Dokument die Schläuche nur durch die außenübergestreifte Hülle in diesen Kerben gehalten, so dass eine Verwendung der Schläuche im Rahmen der dortigen Lehre notwendig die Verwendung auch der Hülle voraussetzt. Im Übrigen geht aus dieser Schrift auch nicht hervor, wie das zweite oben angesprochene Problem der ungewollten Längenänderung zu lösen wäre.

Die Patentschriften US 7,762,949 B2, US 5,944,654, US 4,616,631, US 6,340,344 B1 und US 4,646,722 schlagen jeweils Endoskope vor, in denen der Schaft eine Kerbe aufweist, in die ein flexibler Schlauch einsetzbar ist. In der US 6,340,344 B1 ist die Kerbe an der Seite des Griffstücks fortgesetzt. Lediglich die zeigt hierbei eine Ausführungsform, bei der ein Flächenschwerpunkt einer Komplettierung des Schaftquerschnittes zu einer einfachen Form innerhalb des Schlauches liegt, wobei der Schlauch bei dieser Ausführungsform jedoch im eingesetzten Zustand über diese Schaftkomplettierung hinausragt, so dass der effektive Schaftquerschnitt unvorteilhaft vergrößert ist. Bei allen dort gezeigten Ausführungsformen ist der Schlauch über seine ganze Länge fest in der Kerbe gehalten, so dass eine relative Bewegung von Schlauch und Kerbeninnenwandung nicht möglich ist.

Die Patentschrift US 6,447,445 B1 zeigt eine Endkappe für das distale Ende eines Endoskops, in welchem Arbeitskanäle bereitstellende flexible Schläuche Fixierstrukturen zur Sicherung gegen ein unkontrolliertes Verdrehung aufweisen.

Die Schrift US 7,311,659 B2 schlägt einen Endoskopschaft mit sich spiralig um den Schaft windenden Arbeitskanälen vor, welche in einer gemeinsamen Hülle, und nicht in einer Kerbe geführt sind.

Die Offenlegungsschrift DE 10 2012 105 370 A1 schlägt eine Endoskophülle aus einem lang erstreckten flexiblen Schlauch mit einem Hauptlumen zur Aufnahme zumindest eines Abschnitts eines Endoskopschafts sowie einem oder zwei parallel verlaufenden, sich spiralig um das Hauptumen und damit den Endoskopschaft windenden Arbeitskanälen vor.

In der veröffentlichten europäischen Anmeldung EP 1 284 120 A1 ist ein als Wegwerfprodukt konzipierter Einwegmantel zur Kopplung mit einem Endoskop vorgeschlagen, welcher mindestens einen Endoskopkanal zur Aufnahme des Endoskop(schafte)s sowie dazu parallverlaufende Arbeits-, Spül- und Saugkanäle aufweist.

In der Patentschrift US 4,947,827 werden verschiedene technische Lösungen des Problems der axialen Bewegung eines in eine Kerbe im Endoskopschaft eingesetzten, einen Arbeitskanal für ein Arbeitsgerät bereitstellenden Schlauchs relativ zu den Wänden der Kerbe beim Verbiegen der Endoskopspitze vorgeschlagen, welches daher rührt, dass die Länge der Kerbe sich bei üblichen Endoskopen mit dem Biegungszustand ändert.

Ein dort vorgestellter Lösungsansatz ist die Verwendung eines in axialer Richtung zumindest abschnittsweise flexiblen Schlauchs, wobei diese Flexibilität erreicht wird entweder durch die Verwendung eines flexiblen Materials für die Schlauchwandung, oder die Bereitstellung eines faltenbalgartigen Schlauchabschnitts oder einer Schlauchschleife, welche jedoch jeweils außerhalb der Kerbe angeordnet sein sollen.

Ein alternativer Ansatz versucht, den Endoskopschaft so zu gestalten, dass bei der Verbiegung des Schaftes die oben beschriebene Längenänderung der Kerbe möglichst nicht mehr auftritt. Hierzu müssen die Schnittpunkte der Schwenkachsen der Gelenke der gelenkig miteinander verbundenen Elemente des Endoskopschafts mit der Mittelachse des in die Kerbe eingesetzten Arbeitskanalschlauchs zusammenfallen. Dies kann der dortigen Lehre gemäß entweder dadurch erreicht werden, dass eine ausreichend tiefe Kerbe vorgesehen ist oder es wird eine Kerbe üblicher Tiefe verwendet, jedoch werden besonders gestaltete gelenkig verbundene Elemente eingesetzt, bei denen ein Teil der Gelenke außermittig angeordnet ist, so dass der Schnittpunkt der Schwenkachsen ebenfalls außerhalb der Schaftmitte liegt. Als dritte Möglichkeit nach diesem Ansatz wird vorgeschlagen, den Schaft so zu gestalten, dass eine Verbiegung nur in einer Ebene möglich ist, welche senkrecht zur Verbindungslinie zwischen den Mittenlinien des Schaftes und des Schlauches in der Kerbe steht.

Eine Verbindung dieser Lösungsansätze oder der verschiedenen Ausgestaltungen eines Ansatzes wird nicht vorgeschlagen.

Es ist daher eine Aufgabe der vorliegenden Erfindung, ein Endoskop mit mindestens einem Instrumentier- bzw. Biopsiekanal in seinem Schaft bereitzustellen, welches die Durchführung chirurgischer Eingriffe ein schließlich Biopsien ermöglicht und dabei oben beschriebene Nachteile vermeidet. Insbesondere soll es schnell und einfach zu reinigen und zu sterilisieren sein und dadurch die Zeit zur Vorbereitung des Endoskops zur Wiederverwendung zwischen zwei chirurgischen Eingriffen oder Untersuchungen auch ohne Einsatz einer schützenden Hülle zu reduzieren, wobei gleichzeitig der Schaftdurchmesser des Endoskops nicht größer sein sollte als bei üblichen Endoskopen. Weiterhin sollte die axiale Position eines in den Biopsiekanal eingeführten Instruments so wenig wie möglich durch die Bewegung des Schaftes und seines distalen Endes beeinflusst werden. Ein weiteres Ziel ist es, die oben beschriebenen vorbekannte Endoskope mit Wechselschläuchen dahingehend zu verbessern, dass ein Herausspringen des Schlauches beim Verbiegen des Schaftes im Einsatz zuverlässig verhindert ist, ohne das Einsetzen des Schlauches in seine Kerbe signifikant zu erschweren oder zu verlangsamen.

Als Lösung schlägt vorliegende Erfindung ein zweiteiliges System vor bestehend aus einem Endoskop mit einem Schaft auf dessen Außenseite eine ausreichend tiefe, im Wesentlichen axiale Kerbe verläuft und einem hohlen Arbeits- und/oder Versorgungsschlauch aus einem flexiblen Material zur Bereitstellung eines Instrumenten- bzw. Biopsiekanals, eines Wasserkanals und/oder eines Luft-/Unterdruckkanals, indem der Schlauch, in einem Vorbereitungsschritt vor dem Eingriff oder der Untersuchung, in die Kerbe des Endoskopschaftes eingesetzt wird.

Ein Aspekt vorliegender Erfindung, um diese Ziele zu erreichen, ist ein Endoskop gemäß des Anspruches 1, in dem ein Arbeitsschlauch wieder entfernbar in einer Kerbe eingesetzt ist, welche im Wesentlichen axial an der Außenseite des Schaftes von einer Einbuchtung oder Öffnung in seinem distalen Ende bis zumindest zu einem Punkt nahe des proximalen Endes des Schaftes verläuft. Die Kerbe kann sich auch entlang des gesamten Schaftes von seinem distalen zu seinem proximalen Ende erstrecken und kann darüber hinaus auch nahtlos im Griff, welcher an das proximale Endes des Schaftes angeschlossen ist, weitergeführt sein.

Erfindungsgemäß gibt es entlang des gemeinsamen Verlaufs von Schlauch und Kerbe zumindest einen Abschnitt, in dem beide relativ zueinander hinsichtlich Querschnittsform und -größe derart gestaltet sind, dass der Schlauch in der Kerbe Spiel hat, sich also relativ zu den Innenwandungen der Kerbe leicht verschieben kann, insbesondere in axialer Richtung, aber auch hinsichtlich einer Drehung des Schlauches um die (lokale) Längsachse der Kerbe.

Ein solcher freier Abschnitt des Schlauchs, von dem es auch mehrere geben kann, erlaubt eine bessere Anpassung an Längenänderungen der Kerbe in Folge von Verbiegung des Endoskopschaftes, wie sie beim Einsatz des Endoskops unweigerlich auftreten.

Das Spiel zwischen Schlauch und Kerbe(nwandungen) in dem bzw. einem der freien Abschnitt/e kann insbesondere dadurch erreicht werden, dass, zumindest dann, wenn der Endoskopschaft gerade ausgestreckt ist, der Querschnitt des Schlauchs dort etwas kleiner ist als der Querschnitt der Kerbe. Bei einem Schlauch mit rundem Querschnitt ist dies gleichbedeutend mit einem Außendurchmesser, der kleiner ist als ein Innendurchmesser einer, zumindest teilweise komplementär, etwa U- oder Ω-förmigen geformten Kerbe.

Die zwischen und jenseits des/r freien Abschnitts/e liegenden Abschnitte sind hingegen fester in die Kerbe eingesetzt, entweder die Elastizität des Schlauches ausnutzend eingepresst oder alternativ oder zusätzlich mit einem nicht sehr klebekräftigen Klebstoff eingeklebt.

Um eine Vergrößerung des Querschnittsschaftes zu vermeiden, sind die Formen und relativen Größen der Querschnitte des hohlen Schlauches und der Kerbe derart, dass die geometrische konvexe Hülle oder aber zumindest die Komplettierung des Schaftes die gleiche ist, egal ob der Schlauch in die Kerbe eingesetzt ist oder nicht, in anderen Wort, sofern der Schlauch vollständig in der Kerbe auf der Außenseite des Schaftes untergebracht ist, beispielsweise durch Einstecken oder Eindrücken soweit wie möglich, aber ohne den üblicherweise elastischen Schlauch übermäßig aus seiner natürlicher Form zu deformieren, sollte er an keiner Stelle entlang des Schaftes über die konvexe Hülle oder zumindest die Komplettierung des Schaftes ohne Schlauch hinausragen.

Der Begriff "konvexe Hülle" wird hier mit seiner üblichen mathematischgeometrische Bedeutung verstanden, welches das kleinste konvexe Volumen bezeichnet, welches ein bestimmtes Objekt enthält. "Komplettierung" wird hier verstanden als die gedachte Komplettierung des Querschnitts zu der, flächenmäßig, kleinsten fundamentalen geometrischen Form welche den Querschnitt enthält, wobei als fundamentale geometrische Formen in diesem Zusammenhang Ellipsen (einschließlich Kreise), regelmäßige Vielecke (einschließlich Quadrate) und alle Formen, welche aus einem regelmäßigen Vieleck durch Stauchen bzw. Strecken entlang einer Achse erhältlich sind (dies umfasst Rechtecke) angesehen werden.

Um eine schnelle und einfache Reinigung und Sterilisation des Endes des Endoskops zu erleichtern, sind scharfe innere Kanten in der Kerbe zu vermeiden. Die Innenseite der Kerbe sollte an keinem Punkt einen Krümmungsradius aufweisen, der viel kleiner als der Krümmungsradius eines Querschnitts des hohlen Schlauchs, welcher in ihr untergebracht ist, aufweist, insbesondere Krümmungsradius nicht kleiner als 0,1 mm, ideeller weise nicht kleiner als 0,5 mm.

Eine Kerbe, welche aufgrund ihrer Bemaßung dazu gedacht ist, einen Schlauch für einen Instrumentenkanal zu beherbergen, sollte so angeordnet und dimensioniert sein, dass sichergesellt ist, dass sich die axiale Position eines in den Instrumentenkanal eingeführten Instruments relativ zum Schaft, oder genauer relativ zum distalen Ende davon, beim Verbiegen des Schaftes so wenig wie möglich ändert. Hierfür sollte die Kerbe im Wesentlichen entlang ihrer gesamten Erstreckung einer Linie verschwindender Streckung oder Stauchung des Schaftes nahekommen oder diese idealerweise enthalten, also eine gedachte Linie im Inneren des Schaftes, entlang welcher sich das Material beim Verbiegen des Schaftes weder gestreckt noch gestaucht wird, wenn sich der Schaft beim Einführen in den Patienten, beispielsweise weil der Benutzer des Endoskops Verbiegung des distalen Endes mittels der Kontrolldrähte steuert, ändert.

Dies kann beispielsweise dadurch erreicht werden, dass die Kerbe ausreichend tief, sich beziehend auf ihre radiale Erstreckung, ausgeführt wird, um einen Flächenschwerpunkt entweder des tatsächlichen Querschnitts des Schaftes oder seiner Komplettierung im obigen Sinne nahezukommen oder diese zu enthalten. Die Kerbe kann als dem Flächenschwerpunkt nahe angesehen werden, wenn die Entfernung zwischen Kerbe und Flächenschwerpunkt gleich oder kleiner dem halben Radius des kleinsten Kreises ist, welcher den Querschnitt des Schaftes enthält. Eine häufige Komplettierungsform für Querschnitte von Endoskopschäften gemäß vorliegender Erfindung ist ein Kreis oder eine Ellipse, die einem Kreis nahe kommt, d.h., welche nur eine kleine Exzentrizität aufweist. Der Flächenschwerpunkt dieser Komplettierung ist dann der Mittelpunkt dieses Kreises oder dieser Ellipse.

Ein Aspekt vorliegender Erfindung ist ein hohler Schlauch aus einem flexiblen Material gemäß Anspruch 1 zur Bereitstellung zumindest eines Biopsie- bzw. Instrumentierkanals eines Endoskops mit einer Kerbe entlang der Außenseite seines Schaftes. Der Schlauch hat mindestens ein proximales Ende, worin zumindest eine Öffnung zur Einführung eines chirurgischen Instrumentes, wie beispielsweise einer Biopsiezange an der Spitze eines Führungsdrahtes, und ein distales Ende, mit welchem es in einer Einbuchtung im distalen Ende des Endoskopschaftes gemäß vorliegender Erfindung positioniert wird. Im distalen Ende des Schlauches befindet sich zumindest eine Öffnung, aus der das drahtgeführte chirurgische Instrument die gewünschte Operationsstelle im Inneren des Patienten erreichen kann. Der Schlauch kann auch in seinem Verlauf eine oder mehrere Abzweigungen und hierdurch mehr als ein proximales Ende aufweisen.

Der Schlauch besteht aus einem flexiblen Material, welches sich zusammen mit dem Schaft verbiegen kann und die Positionsänderung des Schaftes, und insbesondere seines distalen Endes, nicht behindert. Sofern ein Material wie beispielsweise synthetisches Gummi oder Polyethylen verwendet wird, welches wegen geringen Herstellungskosten in nahezu jede beliebige Form bringbar ist, wird es wirtschaftlich, den hohlen Schlauch gemäß vorliegender Erfindung als ein wegwerfbares Einwegprodukt zu betrachten. Den Schlauch nach Verwendung des erfindungsgemäßen Endoskops nicht zu reinigen und sterilisieren zu müssen, bedeutet eine deutliche Verringerung der Zeit, welche benötigt wird, das erfindungsgemäße Endoskop wieder einsatzbereit zu machen.

Der Schlauch kann einen kreisförmigen, elliptischen oder ovalen Querschnitt aufweisen, welcher entweder von der gleichen Größe oder etwas größer als der der Kerbe ist. Der Schlauch kann aus einem einzelnen Teil bestehen, kann aber auch aus zwei oder mehr, insbesondere drei oder vier Teilschläuchen bestehen, welche parallel zueinander verlaufen und über dünne Materialbrücken aus demselben Material wie der Schlauch verbunden sind, wobei jeder Teilschlauch für sich genommen einen kreisförmigen, elliptischen oder im allgemeinen ovalen Querschnitt haben kann.

Einen Schlauch aus einen nicht kreisförmigen Querschnitt oder einem mit mehr als einem Teil bestehenden zu verwenden, hat den Vorteil, dass, sofern der Schlauch in einer Kerbe gleichartigen oder im wesentlichen identischen Querschnitt eingesetzt ist, die Winkelausrichtung relativ zum Schaft im Wesentlichen festgelegt ist und sich während der Verwendung des Endoskops, insbesondere während dem Einführen und Repositionieren des Schaftes und der damit einhergehenden häufigen Biegevorgänge in verschiedene Richtungen, nicht ändert.

Zusätzlich oder alternativ zu einem aus parallelen Teilschläuchen bestehenden Schlauch, können auch interne Wände im Schlauchinneren vorhanden sein, welche dieses in zwei oder mehr, insbesondere drei, parallel axial entlang des Schlauches verlaufende Kanäle unterteilt.

Das Innere eines als Biopsie- oder Instrumtierschlauches muss weit genug sein, um die Passage eines chirurgischen Instruments an der Spitze eines Führungsdrahtes zu erlauben. Insbesondere soll es mindestens 1 mm, besser 2,5 mm bis 3,6mm, jedoch nicht weiter als 5 mm sein. Ein Instrumentierschlauch muss damit einen deutlich größeren Innendurchmesser, bzw. eine größere Innenquerschnittsfläche aufweisen, als ein Versorgungsschlauch zur Bereitstellung von Wasser und oder Luft/Unterdruck am Operationsort.

Der erfindungsgemäße Arbeits- und/oder Versorgungsschlauch, insbesondere ein vergleichsweise dickerer Instrumentierschlauch kann faltenbalgartige oder auch spiralschlauchartige Abschnitte aufweisen oder vollständig balgartig bzw. spiralschlauchartig ausgeführt sein um, wie bei einem Strohhalm, die Biegsamkeit zu erhöhen und einen guten Längenausgleich beim Verbiegen- und Verwinden des Endoskopschaftes während des Einsatzes zu gewährleisten.

Die Wände des Schlauches, insbesondere interne Unterteilungswände sollten so dünn wie möglich sein, aber dennoch sicherstellen, dass der Schlauch bei bestimmungsgemäßen Gebrauch nicht leicht reißt oder bricht, insbesondere beim Einsetzen oder Einstecken in die Kerbe des Schaftes eines Endoskops gemäß vorliegender Erfindung oder beim Einführen eines chirurgischen Instruments in den hohlen Schlauch.

Vor dem Einsetzen des Schlauchs in die Kerbe des Schaftes eines Endoskops gemäß der Erfindung kann zunächst eine Endkappe über das distale Ende des Schlauches gesteckt werden. Die Endkappe hat die Funktion, das distale Ende des Schlauches in der Einbuchtung bzw. Öffnung im distalen Ende des Endoskopschaftes zu fixieren. Alternativ oder zusätzlich kann das distale Ende des Schlauches einen etwas größeren Querschnitt als der Rest des Schlauches aufweisen, um die elastischen Spannkräfte, welchen es in der Kerbe bzw. im distalen Ende der Kerbe festhalten, zu erhöhen.

Die Vorbereitung des Endoskops gemäß vorliegender Erfindung für einen Einsatz umfassen im Wesentlichen einzig das Installieren oder Einsetzen des flexiblen hohlen Schlauchs in die Kerbe des Endoskopschaftes und ist wesentlich weniger zeitaufwendig als die Vorbereitung eines Endoskops mit einem bekannten Endoskopschutz in Form einer Schutzhülle.

Eine Methode den Schlauch einzusetzen besteht darin, den Schlauch oberhalb der Kerbe zu positionieren, wobei sein distales Ende mit dem distalen Ende des Endoskopschaftes fluchtet, wobei gegebenenfalls zuerst eine Endkappe aufzuschieben ist, und dann den Schlauch von seinem distalen Ende ausgehend schaftaufwärts durch radiale Kraftausübung händisch in die Kerbe zu drücken, bis der Schlauch entlang ihrer gesamten Länge sicher in der Kerbe sitzt. Abschließend wird gegebenenfalls das proximale Ende eines Instrumentierkanals des Schlauches mit geeigneten Mitteln am Griff befestigt, beispielsweise in dem eine Fixierstruktur dieses proximalen Endes in einer hierfür auf dem Griff vorgesehenen Halterung oder Haltestruktur platziert wird. In ähnlicher Weise werden die proximalen Enden eines ggf. vorhandenen Wasser oder Luftkanals des Schlauches geeignet angeschlossen, beispielsweise an Ventile, die den Fluss von Wasser oder Luft kontrollieren, insbesondere Ventile, die in dafür vorgesehene Haltestrukturen auf dem Griff des Endoskops eingesetzt sind, oder an Ausgänge anderer Geräte, welche die den kontrollierten Fluss von Wasser oder Luft bereitstellen.

Wird der Instrumentierkanal als kombinierter Absaugkanal verwendet muss noch eine vom Hauptkanal abzweigende Weiterführung an eine Unterdruckquelle angeschlossen werden. In diese Weiterführung ist bevorzugt ebenfalls ein Kontrollventil eingeschaltet, welches in eine geeignete, idealerweise ergonomisch positionierte Aufnahme oder Haltestruktur des Griffs eingesetzt wird, damit ein Bediener des erfindungsgemäßen Endoskops einen Absaugvorgang kontrollieren kann, ohne seine Hände vom Griff zu nehmen.

Sofern der oder die Versorgungskanäle für Wasser- und/oder Luft durch separate hohle Schläuche bereitgestellt werden, so können diese in weiterer zu diesem Zweck auf der Außenseite des Schaftes vorhandenen Kerben eingesetzt werden, sofern solche vorhanden sind, oder sie sind zusammen mit dem Instrumentierkanalschlauch in einer einzigen Kerbe eines geeigneten Durchmessers oder eines geeigneten Querschnitts eingesetzt.

Eine Wiederverwendung eines erfindungsgemäßen Endoskops zwischen zwei Untersuchungen oder Operationen am gleichen oder an verschiedenen Patienten umfasst die Schritte des Entfernens des Schlauchs aus der einen oder mehreren Kerben, beispielsweise den Schlauch aus seinem Sitz aus der Kerbe, ausgehend vom proximalen oder vom distalen Ende herausgezogen wird. Der Schlauch oder die Schläuche wird bzw. werden dann entweder gesondert vom Endoskop gereinigt und sterilisiert oder bevorzugt, entsorgt. Das Endoskop selbst wird gewaschen und sterilisiert um sicherzustellen, dass jegliche Kontamination mit Mikroben oder Keimen entfernt oder neutralisiert ist. Dies wird durch das geometrische Design der Kerbe ohne scharfe innere und ebenfalls bevorzugt auch ohne scharfe äußere Kanten erleichtert. Nach der Sterilisation des Endoskops wird dieses wie oben beschrieben durch Einsetzen neuer Arbeits- und/oder Versorgungsschläuche für einen erneuten Einsatz vorbereitet.

Das Alleinstellungsmerkmal des vorliegend vorgeschlagenen Systems aus Endoskop mit gekerbtem Schaft und darin lösbar eingesetztem Schlauch liegt darin, dass auf alle flüssigkeitsführenden oder mit Flüssigkeiten in Berührung kommenden inneren Kanalsysteme verzichtet wird und diese nach außen verlegt werden, wo sie bei der Reinigung und Sterilisation leicht zugänglich sind. Hierbei wird dennoch eine Vergrößerung des Schaftquerschnitts vorteilhaft vermieden und die Längungs- und Knickeinflüsse auf die axiale Position von im Arbeitskanal befindlichen Instrumenten minimiert.

Der große Vorteil des erfindungsgemäßen Systems liegt also darin, dass keine Kanäle im hermetisch abgeschlossenen Inneren des Endoskops bzw. Endoskopschaftes mehr vorhanden bzw. nötig sind. Hierdurch wird zum einen die Geschwindigkeit, aber mehr noch die hygienische Sicherheit bei der Reinigung und Sterilisation dramatisch verbessert. Es sind keinerlei schwer zu erreichende und zu reinigende Räume für Keime wie unsichtbare Schläuche, Ecken, Verbindungen, Kurven, Schlauchoberflächenriefen an den Arbeitskanalinnenseiten mehr vorhanden. Hierdurch kann weiterhin das erfindungsgemäße Endoskop mit wechselbarem Arbeitsschlauch ohne Schutzhülle verwendet werden, was bei uneingeschränktem Kontaminationsschutz eine erhebliche Zeitverkürzung zwischen zwei aufeinanderfolgenden Untersuchungen ermöglicht.

Im Folgenden werden andere bevorzugte Weiterbildungen vorliegender Erfindung, welche einzeln oder in Kombination realisiert werden können, solange sie sich nicht offensichtlich gegenseitig ausschließen, näher vorgestellt.

In manchen Ausführungsformen ist lediglich ein einziger freier Abschnitt vorhanden. Dieser ist dann bevorzugt am Übergang zwischen Endoskopgriff und -schaft oder in etwa der Mitte des Schaftes angeordnet und hat bevorzugt eine Länge von zwischen 5 % - 50 %, besonders bevorzugt 10 %-20 % der Schaftlänge.

Alternativ weisen andere Ausführungsformen des erfindungsgemäßen Endoskops mehrere freie Abschnitte auf, die bevorzugt in gleichmäßigen Abständen über den Endoskopschaft verteilt sind und jeweils eine Länge von bevorzugt zwischen 2 %-20 %, besonders bevorzugt zwischen 5 %-10°% der Schaftlänge haben. Der dem distalen Schaftende nächste freie Bereich hat von diesem bevorzugt einen Abstand, der seiner Länge entspricht.

In den freien Abschnitten hat der Schlauch bevorzugt eine im Vergleich zu den restlichen abschnitten erhöhte Verformbarkeit, wodurch er sich leichter an Deformationen der Kerbe, insbesondere (lokale) Dehnungen oder Stauchungen oder Verdrehungen anpassen kann. Dies kann dadurch erreicht sein, dass der, sowieso elastische, Schlauch hier eine geringere Wandstärke hat. Alternativ oder zusätzlich kann auch ein anderes Material mit einer höheren Elastizität (geringeres Young-Modul) eingesetzt werden.

Eine weitere, alternativ oder zusätzlich einsetzbare Lösung für eine höhere Verformbarkeit ist, den Schlauch in den freien Abschnitten faltenbalgartig zu gestalten, so dass er sich, wie beispielsweise von einem gebogenen Strohhalm bekannt, leicht um einen Faktor 2-3 in axialer Richtung strecken/stauchen kann. Statt oder in Kombination mit einer faltenbalgartigen Ausgestaltung kann auch eine spiralschlauchartige Ausgestaltung gewählt werden. Der Unterschied ist, dass ein Faltenbalg mehrere hintereinanderliegende, nicht zusammenhängende kreisringförmige Einschnürungen bzw. Verdickungen aufweist, während ein Spiralschlauch durch eine zusammenhängende, schraubenförmig den Schlauch umlaufende Einschnürung bzw. Verdickung gekennzeichnet ist.

In wiederum anderen Ausführungsformen ist die Länge des freien Bereichs 100 % der Schaftlänge oder alternativ auch der Länge der Kerbe, sofern diese sich auch im Griff fortsetzt. D.h. der Schlauch hat über die gesamte Länge des Schaftes oder der Kerbe Spiel und kann sich leicht gegenüber den Wandungen der Kerbe leicht verschieben. In diesen Ausgestaltungen kann es aber zu Problem werden, das der Schlauch bei Verbiegung und -drehung des Schaftes aufgrund des Spiels leicht aus der Kerbe springt. Sofern dies bei einer Untersuchung auftritt, müsste das Endoskop wieder herausgezogen und der Schlauch wieder richtig eingesetzt werden. Dem kann dadurch begegnet werden, dass eine Ω-förmige Kerbe mit einem großen Durchmesser-zu-Halsweite-Verhältnis von 2:1 oder mehr verwendet wird.

Damit ein Einsetzen bzw. -pressen des Schlauchs, der leicht wechselbar bleiben soll, nicht zu sehr erschwert wird, wird hierbei empfohlen, das Durchmesser-Halsweite-Verhältnis im Verlauf der Kerbe zu variieren und nur abschnittsweise zur Sicherung des Schlauchs ein großes bis sehr großes Verhältnis vorzusehen

Alternativ oder zusätzlich können auch elastische, in die Kerbe oder den Kerbenhals hineinragende Halteflügel vorgesehen werden. Aufgrund der Elastizität lässt sich der Schlauch mit vergleichsweise geringem Druck einpressen bzw. herausziehen. Er wird während der Verwendung des Endoskops aber sicher durch die Halteflügel in der Kerbe gehalten. Der Vorteil der Halteflügel ist, dass eine Kerbe mit konstantem Querschnitt in den Schaft eingebracht werden kann, was bei der Herstellung einfacher ist, als die vorbeschriebene Kerbe mit variierendem Querschnitt.

Um bei der unvermeidlichen Längenänderungen der Kerbe zu verhindern, dass das distale Ende des Schlauches in die Kerbe zurückgleitet, wird vorgeschlagen, dort eine Rückgleitsicherung vorzusehen. Diese kann die Form einer Scheibe oder eines das distale Schlauchende teilweise umlaufenden Kragens annehmen.

Der Schaft des erfindungsgemäßen Endoskops kann eine einzige Kerbe oder mehrere Kerben der gleichen oder verschiedener Längen, Querschnittsformen und -flächen aufweisen. In einigen Ausführungsformen des erfindungsgemäßen Endoskops hat der Endoskopschaft drei Kerben, von denen einen größer, und insbesondere eine größere Querschnittsfläche aufweist, als die anderen beiden und dazu dient, einen flexiblen hohlen Schlauch zur Bereitstellung eines Instrumentenkanals aufzunehmen.

In bevorzugten Ausführungsformen des Endoskops verläuft die Kerbe zur Aufnahme des hohlen Schlauchs vom distalen Ende des Endoskopschaftes bis zu seinem proximalen Ende. In noch mehr bevorzugten Ausführungsformen wird die Kerbe auch nahtlos im Griff weitergeführt, bevorzugt entlang einer Oberseite des Griffs verlaufend.

Obwohl nicht von kritischer Wichtigkeit, kann zur weiteren Verbesserung der einfachen Reinigung und Sterilisierung, und insbesondere um Schaden an dem vergleichsweise dünnwandigen hohlen Schlauch bei der Installation in der Kerbe zu vermeiden, scharfe äußere Kanten radial nach außen gelegenen oberen Endes der Kerbe in manchen bevorzugten Ausführungsformen der Erfindung verzichtet werden.

Der Querschnitt der Kerbe oder Kerben im Schaft kann u-förmig sein und dadurch eine bessere Zugänglichkeit beim Reinigen des Endoskops ermöglichen. Alternativ oder zusätzlich können einige oder alle Abschnitte der Kerbe Ω-förmig ausgeführt sein, um einen sicheren Sitz des Schlauchs in der Kerbe, insbesondere bei Bewegung und Verbiegung des Schaftes zu verbessern.

Die Bauch-Hals-Relation, also das Verhältnis der größten und der kleinsten Breite einer Ω-förmigen Kerbe sollte nicht allzu viel größer als 1,0 (was einer u-förmigen Querschnittsform entspricht) gewählt sein, um das Einstecken oder Pressen des Schlauchs in seinen Sitz in der Kerbe nicht zu schwierig und potentiell schädigend für den Schlauch sein zu lassen. Auch gilt, dass je größer die Bauch-Hals-Relation ist, desto schwerer ist die Innenseite der Kerbe beim Reinigen zugänglich. Daher ist eine Bauch-Hals-Relation einer Ω-förmigen Kerbe gemäß der Erfindung bevorzugt nicht größer als 2,0 und liegt idealer Weise zwischen 1,1 und 1,5.

In manchen Ausführungsformen hat die Kerbe außerhalb der erfindungsgemäßen freien Bereiche eine größere Bauch-Hals-Relation als in den freien Bereichen. Insbesondere kann dort die Bauch Hals-Relation auch einen Wert von 2,0 oder höher annehmen, um die Fixierung des, sich in den freien Bereichen relativ zur Kerbe beweglichen Schlauches, außerhalb der freien Bereiche zu gewährleisten.

In einigen Ausgestaltungen des erfindungsgemäßen Endoskops hat die Kerbe einen Querschnitt, welcher sich entlang ihres Verlaufs ändert. Dies kann eine Änderung des Durchmessers, d. h. der Breite betreffen, wobei diese insbesondere am oder nahe dem distalen Ende des Schaftes und bevorzugt zumindest einer weiteren Region nahe oder am proximalen Ende der Kerbe kleiner als im Durchschnitt sind. Alternativ oder zusätzlich kann der Querschnitt auch zwischen verschiedenen Formen variieren, beispielsweise zwischen einer Ω-Form in manchen Abschnitten und einer u-Form in anderen. In bevorzugten Ausführungsformung hat die Kerbe beispielsweise in zumindest zwei Abschnitten eine Ω-Form, einer dieser Abschnitte befindet sich bevorzugt nahe dem distalen Ende des Schaftes und der andere nahe dem proximalen Ende der Kerbe, wohingegen sie in anderen Abschnitten u-förmig ist. Somit werden die Vorteile des sicheren Sitzes bei Bewegungen des Schaftes des Ω-förmigen Querschnitts mit der verbesserten Zugänglichkeit beim Reinigen eines u-förmigen Querschnitts kombiniert.

Eine Optimierung hin zu einem noch weiter verbesserten Sitz kann dadurch erreicht werden, dass mehr und/oder längere Ω-förmige Abschnitte verwendet werden, eine Optimierung hin zu besserer Reinigbarkeit durch weniger oder kürzere Ω-förmige Abschnitte erreicht wird. Ein guter Kompromiss scheint darin zu besteht, zwischen zwei und fünf Ωförmige Abschnitte, welche jeweils eine Länge von zwischen zwei und 20 mm aufweisen, einzusetzen.

Bevorzugt sollte der Querschnitt des Schlauchs und der Kerbe bzw. für im Wesentlichen kreisförmige Schläuche und zumindest sektionale kreisförmige Kerben ihr Durchmesser, von gleicher Größe sein, so dass die Außenseite des Schlauchs, wenn er in die Kerbe eingesetzt ist, bündig mit der Außenseite des Endoskopschafts abschließt. D. h., mathematisch gesprochen, der Schlauch sollte die konvexe Hülle bzw. die Komplettierung des Endoskopschaftes berühren. Hierdurch wird erreicht, dass die Außenfläche des Schaftes mit eingesetztem Schlauch so glatt wie möglich ist, was das Einführen des Endoskops in den Patienten erleichtert und diesem so wenig Schmerz wie möglich zuführt. Weiterhin macht eine solche Größenabstimmung das Eindringen von Körperflüssigkeiten und in dieser vorhandenen Kontaminationen in den Zwischenräumen zwischen Schlauch und Kerbe schwieriger.

Den letzten Effekt weiter zu verbessern, wird es von vorliegender Erfindung noch mehr bevorzugt, wenn der hohle Schlauch einen Querschnitt aufweist, der dem Querschnitt der Kerbe soweit als möglich in Form und Größe entspricht. Insbesondere sollte der Querschnitt des Schlauches derart gewählt sein, dass, wenn er vollständig in die Kerbe eingesetzt ist, innerhalb Toleranzen gleich der konvexen Hülle oder der Komplettierung des Schaftes im Sinne obiger Definitionen ist.

Eine bevorzugte Ausführungsform des Schlauchs zur Verwendung mit einem Endoskop, welches gemäß der Erfindung eine Kerbe in seinem Schaft zur Ausnahme eines hohlen Schlauches aufweist, hat einen kreisförmigen Querschnitt mit internen Unterteilungswänden, welche den Innenraum des Schlauches in zwei oder drei Kanäle unterteilen. Ein größerer Kanal dient hierbei dem Führen eines chirurgischen Instruments (Instrumentenkanal oder Biopsiekanal) und gleichzeitig der Absaugung von Flüssigkeiten, und einer oder zwei weiterer kleinerer Kanäle von jeweils ähnlichen oder gleichen Durchmesser, dienen dem Transport von Wasser oder (Druck-)Luft.

In einigem Ausführungsformen vorliegender Erfindung mit zwei oder drei Kanalschläuchen zweigt der Instrumentenkanal in einer Y-Abzweigung von den anderen beiden Kanälen ab und wird als separater Instrumentenkanalschlauch weitergeführt, welcher unter einem gewissen Winkel zur axialen Richtung des Endoskopschafts zu einem proximalen Ende geführt ist, welches am Griff befestigt ist, wohin gegen der oder die anderen Kanal/Kanäle von der Abzweigung aus als zweiter separater Schlauch für einen Wasser und/oder (Druck-)Luftkanal weitergeführt sind. Dieser zweite separate Schlauch kann in der Kerbe weitergeführt sein und ihren axialen Verlauf entlang des Endoskopschafts folgen.

In anderen Ausführungsformen vorliegender Erfindung mit einem Zwei- oder Dreikanalschlauch ist es der Wasser und/oder Luftkanal bzw. -kanäle, der oder die vom Instrumentierkanal abzweigen und es ist der Instrumentierkanal, der gradlinig fortgesetzt weiter in der Kerbe bis zu seinem proximalen Ende verläuft. In diesen Ausführungsformen setzt sich die Kerbe bevorzugt nahtlos im Griff fort und läuft dort bis zu dessen proximalen Ende, wo Mittel zum sicheren Befestigen des proximalen Endes des Instrumentierkanals vorhanden sind. Der Wasser- und/oder Luftkanal bzw. -kanäle zweigen, wie oben erwähnt, auf halben Weg entlang der Kerbe vom Instrumentenkanal ab, bevorzugt im Bereich des distalen Endes des Griffes oder des proximalen Endes des Endoskopschaftes, und werden bevorzugt zu Haltevorrichtungen für Ventile geführt, die den Fluss von Wasser und/oder Luft im Wasser und/oder Luft-/Unterdruckkanal für den Benutzer des Endoskops regulierbar machen.

Um durch den Wasserkanal geleitetes Wasser zur Spülung der Optik im distalen Ende des Endoskopschafts zu verwenden, sollte die winkelmäßige Orientierung des distalen Schlauchendes im distalen Ende der Kerbe derart sein, dass der Wasserausgang des Wasserkanals so nah wie möglich bei der Optik liegt. Üblicherweise bedeutet dies, dass der Wasserkanalausgang in radialer Richtung näher am Zentrum des distalen Endes des Endoskopschaftes zu liegen kommen sollte, als die anderen beiden Ausgänge des Luft-/Unterdruck bzw. des Instrumentierkanals. Dies bedeutet auch, dass es weiter von oberen Öffnungen der Kerbe entfernt zu liegen kommen sollte. Jedoch ist in den Ausführungsformen vorliegender Erfindungen mit einem Multikanalschlauch, bei dem Wasser- und/oder Luftkanal vom Instrumentierkanal abzweigen, welcher gradlinig weiter in der Kerbe geführt ist, diese Abzweigung bevorzugt radial aufwärts oder tangential zu einer Seite, beispielsweise zu der Seite, an welcher die Ventile zur Kontrolle des Flusses von Luft und/oder Wasser befestigt sind, gerichtet. Um dies zu erleichtern ohne den sicheren und festen Sitz des Schlauchs in der Kerbe im Bereich der Abzweigungen zu gefährden, legt vorliegende Erfindung vor, dass die Kanäle im Inneren des Multikanalschlauchs zwischen seinem distalen Ende und dem Abzweigpunkt sich um einen gewissen Winkel umeinander winden, insbesondere einen Winkel zwischen 0 und 180°, bevorzugt zwischen 20 und 90°, noch mehr bevorzugt zwischen 30 und 60°.

Alternativ oder zusätzlich kann auch ein Schlauch mit einem kleineren oder ein Schlauch ohne Verwindungswinkel in Verbindung mit einer Ausführungsform des Endoskops verwendet werden, bei der die Kerbe zumindest zwischen dem distalen Ende und einem Abzweigpunkt des Schlauches einen spiralförmigen Verlauf auf der Außenseite des Endoskopschafts nimmt, wobei die Spirale insbesondere einen Winkel zwischen 0 und 180°, bevorzugt zwischen 20 und 90°, noch mehr bevorzugt zwischen 30 und 60° abdeckt.

In diesen Ausführungsformen des erfindungsgemäßen Endoskops kann der vorgesehene Abzweigpunkt durch eine Verschmälerung oder Reduktion des Querschnitts der Kerbe markiert sein, da von diesem Punkt an unter Umständen noch der Instrumentenschlauch in ihr aufgenommen zu werden braucht, und dieser einen kleineren Durchmesser bzw. kleinere Querschnittsfläche hat als der vollständige Multikanalschlauch zwischen dem distalen Ende und dem Abzweigpunkt.

Um sicherzustellen, dass die Winkelausrichtung im Falle eines Mehrkanal-Arbeitsschlauchs mit kreisförmigem Außenquerschnitt sich nicht während der Verwendung des Endoskops verändert, wird vorgeschlagen ihn mit einer Fixiernase, also einem radialen Vorsprung am oder in der Nähe des distalen Schlauchendes und bevorzugt mindestens einer weiteren Stelle entlang des Schlauches zu versehen. Die Fixiernase oder -nasen sollten eine Breite haben, die der Breite der Kerbe an der Position der Nase entspricht. Falls, insbesondere ein Endoskop mit einer zwischen einer Ω- und einer U-Form variierenden Kerbe verwendet wird, d. h. eine Kerbe mit einem teilweise kreisförmigen Querschnitt mit oder ohne als am oberen, radial außengelegenen Seite, sollte die Breite der Fixiernase die Breite der Öffnungen der Kerbe (u-förmige Kerbe) oder des Halses (Ω-förmige Kerbe) entsprechen.

In alternativen bevorzugten Ausführungsformen des erfindungsgemäßen Systems aus Endoskop und wechselbarem Schlauch besteht letzterer aus mehreren, insbesondere drei oder vier, parallelen Teilschläuchen, welche über dünne Brücken miteinander verbunden sind. Die Brücken bestehen bevorzugt aus dem selben (Kunststoff-)Material, wie die Teilschläuche und sind integral, also einstückig mit diesen. Der Vorteil dieser Ausführungsform ist, dass keine Abzweigungen an genau festgelegten Punkten vorgesehen werden müssen, da die Teilschläuche einfach mittels Durchtrennen der Brücken an jedem beliebigen Punkt oder in jedem beliebigen Bereich voneinander separierbar sind. Dies erleichtert die Verwendung in Endoskopen mit unterschiedlichen Längenmaßen (Schaftlänge, Grifflänge, Distanz Endoskopspitze zu den Abzweigungspunken der Teilschläuche) enorm, ohne dass die Vorbereitungszeit des Endoskops vor einer Verwendung messbar erhöht wäre.

Besonders bevorzugt sind Ausführungsformen mit drei oder vier Teilschläuchen. Eine Ausführungsform mit drei Teilschläuchen umfasst beispielhaft einen zentralen Teilschlauch mit einem ausreichend großen Durchmesser, so dass er als Instrumentenschlauch dienen kann. Mit diesem zentralen Instrumenten-Teilschlauch sind zwei seitlich verlaufende, bevorzugt symmetrisch angeordnete, Teilschläuche mit kleinerem Durchmesser über dünne, d.h. höchstens etwa die Wandstärk der Teilschläuche aufweisende, Materialbrücken verbunden. Die kleineren Teilschläuche dienen hier der Bereitstellung eines (Druck-)Luft und eines Wasserkanals

In einer Modifikation dieser Ausführungsform kann der Instrumentierkanal-Teilschlauch auch gedoppelt werden, so dass zwei parallele Instrumentierkanäle vorhanden sind, welche übereinander oder auch nebeneinander angeordnet sein können. Dies erlaubt das gleichzeitige Einführen und Arbeiten mit zwei Instrumenten, welches in manchen Situationen eine Operation beschleunigt oder auch erst möglich macht.

Damit das Endoskop bei einer Operation richtig bestimmungsgemäß verwendbar ist, müssen die proximalen Enden der einzelnen Kanäle, d.h. des Instrumentier- und ggf. kombinierten Absaugkanals, des Wasser- und des Luftkanals, entsprechend gesichert oder angeschlossen werden. Das proximale Ende des Instrumentierkanals muss am Griff des Endoskops befestigt werden, wohingegen die proximalen Endes des Wasser- und Luft-/Unterdruckkanals mit einer Quelle des kontrollierten Flusses von Wasser und Luft (bereitgestellt mit einem Über- oder Unterdruck) angeschlossen werden.

Eine einfache Möglichkeit das proximale Ende des Instrumentierkanals zu befestigen, wäre, es am Griff mit einem Draht oder einer Schnur festzubinden. Dies ist jedoch weder sonderlich sicher noch einfach schnell zu erledigen. Es wird daher von vorliegender Erfindung vorgeschlagen, das proximale Ende des Instrumentierkanals als eine Fixierungsstruktur wie beispielsweise einem quader- oder scheibenförmigen Block zu gestalten und hierfür eine geeignete Haltestruktur zur Festlegung dieses Fixierstruktur am Griff, bevorzugt auf seiner Oberseite bereitzustellen, um einen Zugang zum proximalen Ende zu erleichtern. Die Haltestruktur sollte in eine Höhlung von einer zur Fixierungsstruktur komplementären Form aufweisen. Beispielsweise kann die Haltestruktur aus zwei u-förmigen Schienen, welche parallel zueinander einander gegenüber angeordnet sind und in deren Zwischenraum die Fixierungsstruktur, insbesondere ein quaderförmiger oder scheibenförmiger Block durch Einschieben von oben eingesetzt werden kann. Eine Verjüngung in dem Abstand der Schienen oder der Breite der einzelnen Schienen oder alternativ oder zusätzlichen dem quader- oder scheibenförmigen Block oder beides stellt ein Verstärken der elastischen Anpresskraft sicher, welche die Fixierungsstruktur an ihrem Platz hält.

Alternativ oder zusätzlich können am oberen Ende der Fixierungsstruktur eine Stufe oder eine Vergrößerung in axialer Richtung vorgesehen sein, welche ebenfalls dazu dient die Klemmkraft zu erhöhen. Andere Wege, die Fixierungsstruktur sicher zu befestigen, sind dem Fachmann geläufig.

Eine Steuerung des Flusses von Wasser und/oder Luft und/oder einer Absaugung durch einen durch den Verwender des Endoskops kann dadurch erreicht werden, dass hierfür Ventile in die entsprechenden Kanäle eingeschaltet werden, welche an einem mit den Händen während einer Operation einfach zu erreichenden Ort platziert sind. Insbesondere können die Ventile in Haltevorrichtungen am Griff des Endoskops installiert bzw. eingesetzt sein. Die Ventile können als separate Teile, an welche das proximale Ende der jeweiligen Schläuche anzuschließen ist, bereitgestellt werden. Alternativ, und um die Vorbereitungszeit weiter zu reduzieren, schlägt vorliegende Erfindung vor, die Ventile in die Schläuche zu integrieren, beispielsweise sie im Schlauch vorzuinstallieren. Um die Befestigung der Ventile am Griff zu erleichtern wird weiterhin vorgeschlagen, die Ventile zumindest teilweise in Ventilblöcken einzuschließen, z. B. Ventilblöcke, welche aus dem gleichen Material wie der Schlauch hergestellt sind und Form eines Quaders oder Zylinders haben, welche während der Vorbereitung des Endoskops einfach und schnell in entsprechend geformte Haltevorrichtungen am Griff geschoben werden können.

Der Querschnitt des Endoskopschaft gemäß vorliegender Erfindung kann in bevorzugten Ausführungsformen zumindest im Wesentlichen entlang seiner gesamten Länge spiegelsymmetrisch sein. Insbesondere kann er kreisförmig, elliptisch oder oval sein. Um sicherzustellen, dass die Kerbe aber möglichst nahe an einer Linie verschwindenden oder minimalen Zerrung/Stauchung liegt, kann in diesen Ausführungsformen die Kerbe in einer Symmetrieebene des Endoskopschaftes liegen.

In bevorzugten Ausführungsformen des Endoskops vorliegender Erfindung ist der Schaft nicht, wie im Stand der Technik üblich, als eine kunststoffumhüllte metallische Spiralfeder ausgebildet sondern aus Vollmaterial gefertigt. Insbesondere bei der Verwendung eines Vollkunststoffschaftes hat dies den großen Vorteil, dass durch die Materialwahl ein besserer Kompromiss zwischen Steifigkeit auf der einen und Flexibilität auf der anderen Seite gefunden werden kann. Eine gewisse Steifigkeit ist nötig, um die Richtungssteuerbarkeit der Endoskopspitze beim Einführen in den Körper unter dem Einfluss des ablenkenden Widerstands von Gewebe und Organen zu gewährleisten. Anderseits darf die Steifigkeit natürlich auch nicht so hoch sein, dass ein Verletzungsrisiko besteht. Bei den üblichen Endoskopschäften mit einer Spiralfeder als formgebender Struktur ist die Steifigkeit aber recht niedrig, was das Ansteuern des gewünschten Operations- oder Untersuchungsortes im Patienten erschwert.

Die Verwendung eines Vollmaterialschaftes in Verbindung mit einem mit Arbeitskanälen ausgestatteten Endoskop wird jedoch erst im Rahmen vorliegender Erfindung überhaupt erst möglich, denn aufgrund der Materialermüdung würden die Arbeitskanäle eines Vollmaterialschafts im Laufe der Zeit beschädigt und unbenutzbar werden. Durch das vorliegend vorteilhaft vorgeschlagene Endoskop mit extern zugänglichen, entfernbaren Arbeitskanälen ist diese Einschränkung überwunden, da die Arbeitskanäle und die diese bereitstellenden Schläuche nach jeder Verwendung des Endoskops gewechselt werden.

Ein Vollmaterialschaft ist allerdings kaum mit einer Steuerung mittels eines innenliegenden Bowdenzugsystems kombinierbar. Daher wird vorgeschlagen, in das Vollmaterial Hydraulikleitungen einer hydraulischen Steuerung einzuschließen, bzw., je nach Herstellungsverfahren des Schaftes, einzugießen. Die Bedienung, also die Steuerung des auf die einzelnen Leitungen beaufschlagten Hydraulikdrucks, würde vom Benutzer in grundsätzlich bekannter Weise durch im Griff in ergonomisch günstiger Position eingelassene Wippschalter anstelle der bei Bowdenzugsteuerungen üblichen Drehräder vorgenommen. Beleuchtung und optische Überwachung könnten weiterhin entweder mittels optischer Fasern oder elektrisch angesteuerter Elemente (Kamera, LED) realisiert werden.

Alternativ oder zusätzlich zur Sicherstellung eines festen Sitzes des Schlauchs in der Kerbe durch rein elastische Kräfte wie im Falle eines Presssitzes u-förmigen Kerbe mit einem Schlauch größeren Durchmessers, oder eine Kombination aus elastischen Kräften und/oder geometrischer Beschränkung, wie im Fall eines zumindest teilweise oder abschnittsweise Ω-förmigen Kerbe, kann ein schwacher und leichtentfernbarer Klebstoff verwendet werden. Er kann zum Zeitpunkt des Einsetzens des Schlauchs in die Kerbe während der Vorbereitung des Endoskops aufgetragen werden oder er wird bei der Herstellung des Schlauchs voraufgetragen an einer oder einer kleinen Zahl von Stellen oder überall über denjenigen Oberteilen des Schlauchs erteilt aufgetragen werden, welcher mit den Innenwänden der Kerbe in Berührung kommt.

In weiteren bevorzugten Ausführungsformen des erfindungsgemäßen Endoskops ist eine Kontaminationsschutzhülle vorgesehen, welche die Reinigung des Endoskops nach dem Einsatz noch weiter erleichtert.

In einer einfachen Ausführungsform wird vor dem Einsetzen des erfindungsgemäßen Schlauchs eine an sich bekannte Schutzhülle über den Endoskopschaft aufgezogen. Solche Schutzhüllen bestehen üblicherweise aus einem hochelastischen Material wie Latex oder Nitrilkautschuk und nehmen daher, vor dem Einsetzen des Schlauches, einen Querschnitt an, welcher nahezu der konvexen Hülle des Schaftes entspricht. Der erfindungsgemäße Schlauch wird nun wie oben beschrieben in die Kerbe eingesetzt, wodurch die Hülle in die Kerbe gedrückt und zwischen der Mantelfläche des Schlauches und der Innenwandung der Kerbe eingeklemmt wird. Diese Klemmung ist stärker, d.h. kraftvoller, in den Bereichen, in denen der Schlauch fest in der Kerbe sitzt und schwächer in den erfindungsgemäßen freien Bereichen des Schlauches.

In einer Abwandlung kann eine zweite, äußere Schutzhülle über die Innere Hülle und den Schlauch geschoben werden. Hierdurch lässt sich ein Kontaminationsschutz nicht nur für das Endoskop selbst, sondern auch für den Bediener realisieren, wenn die äußere Hülle am proximalen Ende entsprechend aufgeweitet und so befestigt wird, dass der Bediener nicht mit nach dem Herausziehen am Endoskop haftenden Körperflüssigkeiten in Berührung kommt.

In einer besonders bevorzugten Ausgestaltung wird eine Schutzhülle verwendet, bei der der Schlauch bereits in die Hülle integriert ist. Beim Aufziehen der Hülle, welches bevorzugt derart geschieht, das die torusförmig aufgerollte Hülle auf die Schaftspitze aufgesetzt und dann kondomartig entlang des Schaftes abgerollt wird, wird gleichzeitig der Schlauch in die Kerbe eingepresst. Hierdurch können Hülle und Schlauch gleichzeitig installiert werden, was die Vorbereitungszeit für eine Untersuchung weiter vorteilhaft verkürzt.

Um die Optik des Endoskops nicht zu behindern ist in der Spitze der Hülle ein Fenster aus einem transparenten Material integriert. Alternativ kann auch die gesamte Hüllenspitze aus einem transparenten Material gefertigt sein. Da die Hülle im Bereich der Spitze nicht so stark gedehnt werden muss, kann auch ein deutlich weniger elastisches Material als für den Rest der Hülle eingesetzt werden.

Es folgt ein kurze Beschreibung der Zeichnungen:
- Figur 1A - D:: Verschiedene Ansichten des distalen Endes des Schaftes einer ersten bevorzugten Ausführungsform des erfindungsgemäßen Endoskops mit einer einzelnen □-förmigen Kerbe in welche ein Dreikanalschlauch eingesetzt ist.
- Figur 2A - D:: Verschiedene Ansichten des distalen Endes des Schafts einer zweiten bevorzugten Ausführungsform des Endoskops gemäß vorliegender Erfindung mit drei separaten Kerben, eine tiefe, leicht □-förmige Kerbe für einen Instrumentenschlauch und zwei kleinere zur Aufnahme eines Wasser- respektive Luftkanalschlauchs.
- Figur 3A:: Schematische perspektivische Ansicht des Griffs einer bevorzugten Ausführungsform der Erfindung mit einem Zugang zum Instrumentenkanal am proximalen Griffende, wobei der Griff weiterhin Haltestrukturen zum Einsetzen von Ventilen, welche an ein Wasser- und/oder Luft-/ Unterdruckkanal angeschlossen oder integriert sind, aufweist.
- Figur 3B:: Schemazeichnung des Verlaufs der Kanäle in der Ausführungsform des erfindungsgemäßen Endoskops nach Figur 3A in der die Kanäle für Wasser- und Luft- vom Instrumentenkanal abzweigen und der Instrumentenkanal als separater Schlauch bis zum proximalen Griffende geführt ist.
- Figur 4A:: Schematisch perspektivische Ansicht des Griffs einer weiteren bevorzugten Ausführungsform vorliegender Erfindung ähnlich der aus Figur 3a, jedoch mit Zugang zum Instrumentenkanal nahe dem distalen Griffende.
- Figur 4B:: Schematische Zeichnung des Verlaufs der Kanäle in der Ausführungsform des Endoskops gemäß vorliegender Erfindung aus Figur 4a, in der der Instumentenkanal vom Wasser- und Luftkanal abzweigt.
- Figur 5A, B:: Perspektivische Ansicht des Schaftes und seines distalen Endes einer dritten Ausführungsform des Endoskops gemäß vorliegender Erfindung in der ein dreiteiliger Schlauch verwendet wird.
- Figur 6A, B:: Perspektivische Ansichten des distalen Endes eines Schlauches gemäß vorliegender Erfindung der aus drei Teilschläuchen besteht, welche parallel zueinander verlaufen und miteinander über dünne Brücken verbunden sind.
- Figur 7A-C:: Perspektivische Ansicht des proximalen Endes des Schaftes und des distalen Endes des Griffs einer Ausführungsform des Endoskops gemäß vorliegender Erfindung welche den dreiteiligen Schlauch aus Figur 6 einsetzt und in der die Abzweigung des Biopsie- und Wasser- und Luft-/Unterdruckkanals gezeigt ist.
- Figur 8A:: Schematischer Längsschnitt durch den Griff einer weiteren Ausführungsform des erfindungsgemäßen Endoskops, bei welcher Instrumentierkanal über zwei proximale Enden mit Öffnungen zum Einführen eines Instruments verfügt.
- Figur 8B:: Rückansicht der Ausführungsform aus Figur 8A (ebenso Figur 9)
- Figur 9:: Schematischer Längsschnitt durch den Griff der Ausführungsform des erfindungsgemäßen Endoskops nach Figur 4.
- Figur 10A,B:: Eine Ausführungsform des erfindungsgemäßen Endoskops mit einem Schaft aus Vollmaterial.
- Fig. 11:: Vorderansicht des distalen Endes einer weiteren Ausführungsform des erfindungsgemäßen Endoskops mit zwei Instrumentenschlauch-Kerben.
- Fig. 12A, B:: Querschnitte durch zwei Ausführungsformen des erfindungsgemäßen Schlauchs bestehend aus vier Teilschläuchen mit doppeltem Instrumentenschlauch.
- Fig. 13:: Ausführungsform des erfindungsgemäßen Endoskops mit zwei Instrumentenkanälen, wobei die Zugangsöffnung des einen distal- die des anderen proximal am Griff angeordnet ist.
- Fig. 14:: In zwei Teilfiguren Ansichten des distalen Schaftendes einer Ausführungsform des erfindungsgemäßen Endoskops mit elastischen Halteflügeln.
- Fig. 15A:: Ansichten des distalen Endes eines erfindungsgemäßen Schlauchs mit internen Unterteilungen in Ausführungsformen mit einer scheibenförmigen Verdickung als Rückgleitsicherung im Vergleich zu einer Ausführungsform ohne Rückgleitsicherung.
- Fig. 15B:: Zwei Ansichten des distalen Endes eines erfindungsgemäßen Schlauchs mit internen Unterteilungen in Ausführungsformen mit einer Kunststoff-Ringscheibe als Rückgleitsicherung in Verbindung mit einer Fixiernase.
- Fig. 16:: Perspektivische Ansicht einer hydraulisch betätigen Ausführungsform eines erfindungsgemäßen Endoskops.
- Fig. 17A:: Vorderansicht einer Kontaminationsschutzhülle mit einem integrierten Schlauch zur Verwendung mit einem erfindungsgemäßen Endoskop.
- Fig. 17B:: Aufgeschnittene perspektivische Ansicht der Hülle aus Fig. 17A.
- Fig. 17C:: Querschnitt durch die Schaftspitze einer bevorzugten Ausführungsform eines erfindungsgemäßen Endoskops, auf welches die Hülle der Figuren 17A und 17B augezogen ist.
- Fig. 18:: Querschnitt durch die Schaftspitze der bevorzugten Ausführungsform eines erfindungsgemäßen Endoskops nach Fig. 17C, jedoch mit zwei übereinander auf den Schaft aufgezogenen Schutzhüllen und einem separaten, im Zwischenraum zwischen den Hüllen angeordneten, in die Kerbe des Schaftes eingesetzten Schlauch.

Im Folgenden werden bevorzugte Ausführungsformen des Endoskops gemäß vorliegender Erfindung unter Verweis auf die Figuren im Detail beschrieben. Diese Ausführungsformen sollen den Gegenstand vorliegender Erfindung lediglich illustrieren und in keinster Weise einschränken. Merkmale können verändert und Merkmale die im Kontext in verschiedenen Ausführungsformen gezeigt sind miteinander kombiniert werden ohne die Idee und den Bereich vorliegender Erfindung zu verlassen.

In den Figuren bezeichnen gleiche Bezugszeichen Merkmale oder Strukturen gleicher Funktionen oder gleicher Bedeutung.

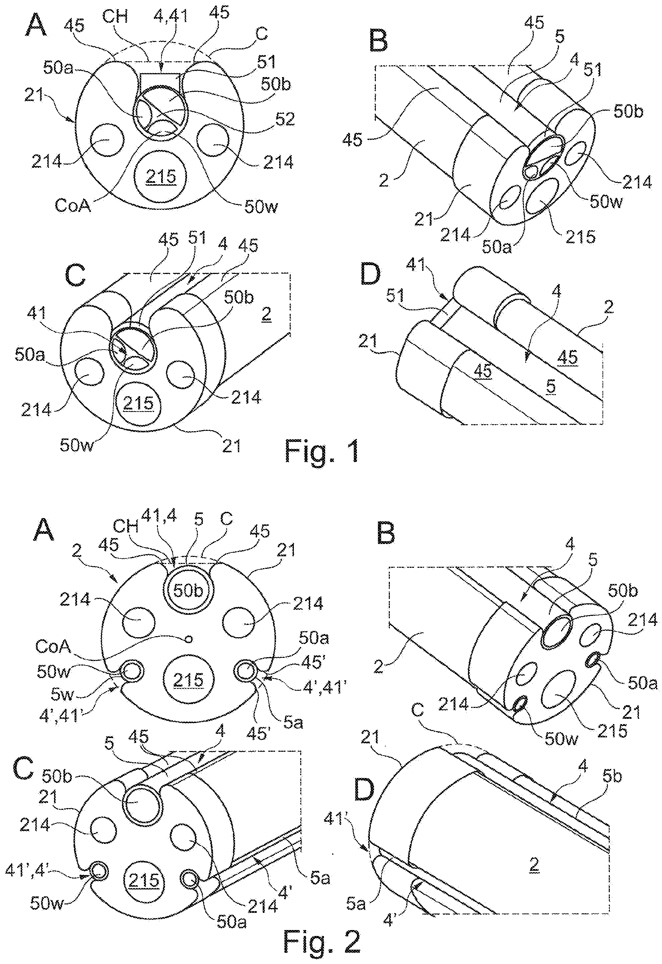

Eine erste bevorzugte Ausführungsform des erfindungsgemäßen Endoskops ist in **Figur** 1 gezeigt, wo vier verschiedene Ansichten des distalen Endes des Endoskopschaftes zu sehen sind.

**(Teil-)****Figur 1A** zeigt eine Vorderansicht des distalen Endes 21 des Endoskopschaftes 2. Zu erkennen sind das distale Ende 41 der Kerbe 4 welche den hohlen Schlauch 5 beherbergt, welcher in dieser Ausführungsform interne Wände 52 zur Unterteilung in drei Kanäle hat: ein größerer Instrumentenkanal 50b, der auch als Absaugkanal verwendbar ist, und zwei kleinere Kanäle, einen Kanal 50a, der dazu dient zur Insufflation (Druck-)Luft bereitzustellen und einen Kanal 50w der zum Zweck der Reinigung und Spülung von Operationsstelle, der Endoskopoptik oder des Instruments Wasser führt. Die Winkelausrichtung des außen im Wesentlichen kreisförmigen Schlauchs 5 ist derart, dass der Auslass des Wasserkanals 50w der optischen Linse 215 im distalen Ende 21 möglichst nahe kommt um während der Verwendung des Endoskops die Linse reinigen zu können. Sofern das distale Ende 21 durch Drehen und Verbiegen des Endoskopschafts in die richtige Position gebracht wird, ist das Wasser aus dem Auslass des Wasserkanals 50w derart, dass es auch zur Reinigung der Lichtausgänge 214, welche beidseitig der die Kerbe 4 und die optische Linse 215 verbindende Symmetrielinie angeordnet sind, verwendbar. Die richtige winkelmäßige Ausrichtung des distalen Endes von Schlauch 5 ist durch die Fixiernase 51 sichergestellt, welche eine Rotation des Schlauchs 5 auch im Falle des Verbiegens des distalen Endes des Endoskopschaftes wirksam verhindert.

Die Nase 51 ist auch in den Teilfiguren B - D zu sehen, welche jeweils perspektivische Ansichten des distalen Endes 21 des Endoskopschaftes 2 dieser Ausführungsform der Erfindung zeigen.

Form und Größe des Querschnitts des Schaftes 2 und der Kerbe 4, insbesondere deren radiale Erstreckung bzw. Tiefe, ist am besten in Teilfigur A zu sehen. Dort ebenfalls gezeigt sind die Komplettierung sowie die konvexe Hülle des Querschnitts. Es wird deutlich, dass der Flächenschwerpunkt CoA des Kreises C innerhalb der Kerbe 4 und damit auch innerhalb des Schlauchs 5 liegt. Obwohl dies in der Figur genaugenommen nur für das distale Ende 21 zu sehen ist, gilt dies auch im Wesentlichen für die gesamte Erstreckung der Kerbe 4 entlang des Schaftes 2. Der Flächenschwerpunkt der konvexen Hülle CH ist hier nicht dargestellt, würde jedoch sehr nahe bei dem gezeigten Punkt COA und damit ebenfalls nahe bei oder sogar innerhalb der Kerbe 4 liegen. Die Anordnung und Wahl der Größe von Kerbe 4 stellt sicher, dass es eine Linie verschwindende Dehnung bzw. Stauchung auf ihrer ganzen Länge enthält, was den Vorteil hat, dass die axiale Position eines in den Instrumentenkanal eingeführten Instruments nicht oder nur wenig durch ein Verbiegen des Endoskopschaftes beeinflusst wird.

Die Kerbe 4 in dieser Ausführungsform ist, zumindest in der Nähe des distalen Endes 41, omegaförmig um einen sichereren Sitz des Schlauchs 5 in der Kerbe 4 zu gewährleisten. Um allerdings das Installieren des Schlauches nicht übermäßig zu erschweren oder potentiell tätigend zu gestalten wurde eine moderate Bauch-zu-Hals-Relation von ca. 1,3 gewählt. Jedoch ist irgendein Wert zwischen 1,1 und 1,5 gleichermaßen bevorzugt.

Die Kerbe 4 hat abgerundete Ecken 45 um Schaden zum flexiblen Schlauch 5 während des Einsetzens zu verhindern und auch da scharfe Kanten relativ schnell schartig würden und somit ein Habitat für Mikroben und Keime darstellten, welches schwer zu reinigen wäre. Um Reinigung und Sterilisation zu erleichtern, wurde auf scharfe innere Kanten in der Kerbe 4 ebenfalls verzichtet und sie darüber hinaus ausreichend breit und groß ausgeführt um eine leichte Zugänglichkeit durch Reinigungsbürsten und Düsen von Reinigungsgeräten zu ermöglichen.

Zwar sind der Endoskopschaft 2 und sein distales Ende 21 in dieser und ebenfalls in anderen Ausführungsformen, welche unten dargestellt werden, spiegelsymmetrisch, doch auch dies in der vorlegenden Erfindung im Allgemeinen nicht der Fall zu sein. Eine nicht symmetrische Form kann verwendet werden ohne vom Geist der Erfindung sich zu entfernen. Jedoch hat eine symmetrische Form Vorteile bei der Herstellung und ist auch für ein Verwender des Endoskops leichter zu kontrollieren und zu fühlen.

Die vier Teilfiguren von **Figur** 2 illustrieren das distale Ende des Schaftes einer zweiten bevorzugten Ausführungsform der Erfindung.

Teilfigur A zeigt wieder eine direkte Draufsicht während die Teilfiguren B - D perspektivische Ansichten aus drei verschiedenen Winkeln zeigen.

In dieser Ausführungsform hat der Endoskopschaft 2 drei Kerben: Eine größere Kerbe 4 zur Aufnahme eines Schlauches 5 für einen auch zur Absaugung verwendbaren Instrumentierkanal 50 und zwei kleinere Kerben 4' zur Aufnahme von Schläuchen 5a und 5w, Bereitstellung eines Luftkanals 50a respektive eines Wasserkanals 50w. Alle Schläuche haben einen kreisförmigen Querschnitt. Die Kerben 4 und 4' haben alle jeweils einen omegaförmigen Querschnitt um einen sicheren Sitz der Schläuche 5b, 5a und 5w, in den Kerben sicherzustellen, wobei das Einsetzen durch längsweises Einstecken oder Drücken in die Kerbe geschehen kann. Hier wurde eine im Vergleich zur Ausführungsform von Figur 1 kleinerer Bauch-Hals-Relation von 1,2 gewählt, welche in einen weniger, jedoch immer noch ausreichend sicheren Sitz, dafür aber eine einfachere Installation der Schläuche während des Aufbaus des Endoskops resultiert und auch eine schnellere und leichtere Reinigung aufgrund der besseren Zugänglichkeit der Innenwände der Kerben 4, 4' während des Reinigungs- und Sterilisationsvorgangs nach der Benutzung des Endoskops ermöglicht.

Die Komplettierung des Schaftes 2 dieser Ausführungsform ist wieder ein Kreis C, ähnlich wie in der Ausführungsform nach Figur 1. Obwohl der Flächenschwerpunkt CoA dieses Kreises C nicht in der Hauptkerbe 4 mit Schlauch 5b liegt, ist es immer noch nah an dieser Kerbe 4 in dem Sinne, dass der Punkt CoA innerhalb einer Distanz von weniger als der Hälfte des Radius des Kreises C vom nächsten Punkt der Kerbe 4 entfernt liegt. Daher ist die Zerrung bzw. Stauchung, welche die Kerbe 4 und der Schlauch 5b darin ausgesetzt sind, immer noch vergleichsweise klein und eine ungewünschte axiale Bewegung eines Instrumentes im Instrumentenkanal 50b im Instrumentenkanalschlauch 5b während des Verbiegens des Endoskopschaftes 2 immer noch akzeptabel.

Wie in der ersten Ausführungsform kann das Wasser des Wasserkanals 50w dafür zur Reinigung der optischen Linse 215 verwendet werden. Jedoch kann nur eine der Lichtauslässe 214 auf einfache Art mit Wasser gereinigt werden wohingegen der andere, da er auf der anderen Seite jenseits des Gewichtsschwerpunktes liegt, durch das Wasser aus Kanal 50w schwerer erreicht werden kann.

Ebenfalls ähnlich der Ausführungsform aus Figur 1 sind die äußeren Kanten 45, 45' der Kerben 4, 4' gerundet und scharfe innere Kanten werden vermieden sowie die Breite von Kerbe 4 auch groß genug ist um eine gute Zugänglichkeit während des Reinigens und der Sterilisation zu ermöglichen.

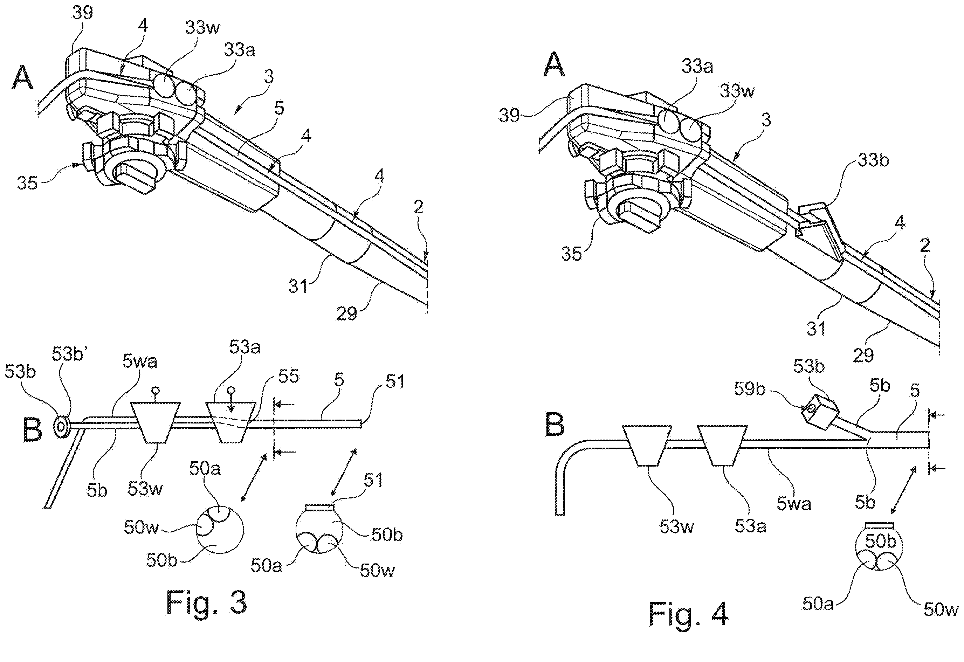

Sowohl in **Figur 3** als auch in **Figur 4** zeigt die Teilfigur A jeweils den Griff und das proximale Ende des Schaftes zweier ähnlicher aber in etwas verschiedener Ausführungsformen des Endoskops gemäß vorliegender Erfindung.

Gemeinsam ist beiden die Form des Griffes mit Kontrollrädern 35 zur Kontrolle der Biegerichtung des distalen Endes des Schaftes 2, wobei die Kontrollräder 35 auf der rechten Seite des Griffs 3 angeordnet sind. Ebenfalls ist in beiden Ausführungsformen die Kerbe 4 auf der Außenseite des Schafts 2 nahtlos auf der Oberseite des Griffs 3 fortgesetzt und erstreckt sich bis zum proximalen Ende des Griffs 3. In der Kerbe 4 ist der Schlauch 5 in einer Weise eingesetzt, dass er während der Verwendung des Endoskops, bei der der Endoskopschaft stark bewegt oder gebogen werden kann, nicht herausfällt. Nahe des proximalen Endes 39 des Griffes 3 befinden sich Haltestrukturen 33a und 33w zur Aufnahme von Ventilen der Ventilblöcke 53a und 53w der jeweiligen Teilfiguren b in dem diese von oben in die Haltestrukturen eingesetzt und bis in eine vollständig versenkte Position eingeschoben werden. Die Kerbe 4 verläuft mittig durch die Haltestrukturen 33a und 33w durch Schlitze in den axialen Stirnseiten der Haltestrukturen.

Die beiden Ausführungsformen aus Figur 3 und Figur 4 unterscheiden sich einmal in der Position des proximalen Endes des Instrumentenkanals 50b. Während die Ausführungsform aus Figur 3 der Instrumentenkanal bis zum proximalen Ende 39 des Griffs 3 geführt ist und damit hinter den Haltestrukturen 33a und 33w für die Ventile zu liegen kommt, hat die Ausführungsform aus Figur 4 eine zusätzliche Haltestruktur 33b zur Befestigung des proximalen Endes des Instrumentenkanals, welche vom proximalen Ende 39 aus gesehen in der Richtung auf das distale Ende zwischen diesem und den Strukturen 33a, 33w positioniert ist (Vergleiche Figur 4A).

Diese unterschiedliche Positionierung des proximalen Endes zieht eine unterschiedliche Gestaltung des Hohlschlauchs 5 in beiden Fällen nach sich, welche in den Teilfiguren 3B und 4B illustriert ist. Wie in Figur 3B gezeigt, ist bei der Ausführungsform, bei der das proximale Ende 59b des Instrumentenkanals 50b mit der Öffnung zum Einschub eines Instruments in der Nähe des proximalen Endes 39 des Griffs 3 positioniert ist, es der Wasser- und/oder der (Druck-)Luftkanal, welcher zuvor mit dem Instrumentenkanal 50b in einem Multikanalschlauch wie in der Bildzeichnung dargestellt vereinigt waren, (vergleiche auch Figur 1), der vom Schlauch 5 in einer vor den Ventilblöcken 53a, 53w gelegenen Y-Abzweigung 55 abzweigt. Von diesem Punkt an verlaufen Instrumentenkanal 50w und Wasser- und Luft-/Unterdruckkanal 50w, 50a in separaten Schläuchen 5b, 5aw, welche bis fast zum proximalen Ende 39 des Griffs 3 weiterhin gemeinsam in der Kerbe 4 untergebracht sind. Y-Abzweigung 55 hat einen kleinen Winkel zwischen beiden Ästen, welche zu Schläuchen 5b und 5aw führen von 15°oder weniger um das gemeinsame Führen beider Teilschläuche 5e und 5aw in der Kerbe 4 zu erleichtern.

Um die Abzweigung des Wasser- und Luftkanals vom Instrumentenkanal zu ermöglichen, winden sich diese um den Instrumentenkanal, wie aus den beiden Teilzeichnungen von (Teil-)Figur 3B hervorgeht. Die rechte zeigt eine Vorderansicht des distalen Endes von Schlauch 5, an welchem zur winkelmäßigen Fixierung in der Kerbe eine Fixiernase 51 angeformt ist. Die Ausgangsöffnungen des Wasserkanals 50w und des Luftkanals 50a sind hierbei am Kerbengrund angeordnet. Im Verlauf des Schlauches winden sich Wasser- und Luftkanal um den Instrumentenkanal, so dass sie im Bereich der Abzweigung 55 oberhalb des Instrumentenkanals zu liegen kommen, wie aus der linken Teilzeichnung, welche einen Querschnitt durch den Schlauch im Bereich vor der Abzweigung 55 zeigt, zu sehen ist. Der Verwindungswinkel zwischen distalem Schlauchende und Abzweigung 55 beträgt hier ca. 120 Grad, wobei im Allgemeinen ein Verwindungswinkel zwischen 0 und 180, möglich ist, aber Winkel zw. 30 und 150 Grad, insbesondere 60 und 120 Grad, bevorzugt sind.

In der Ausführungsform nach Figur 4, wo das proximale Ende des Instrumentenkanals 50b mit der Einführungsöffnung vor d.h. in Richtung auf das distale Ende 31 des Griffs 3 von den Haltestrukturen 33 aus gesehen liegt, zweigt der Instrumentenkanal 50b ebenfalls als ein separater Schlauch 5b in einer Y-Abzweigung 56 ab, wobei diese Abzweigung ein wesentlich höheren Winkel von ca. 30° zwischen den beiden abzweigenden Ästen einnimmt, um den Zugang zu der Einführungsöffnung im proximalen Ende 59b zu erleichtern, wenn dieses in die Haltestruktur 33b eingesetzt ist.

In beiden Ausführungsformen ist das proximale Ende 59b des Instrumentenkanalschlauchs 5b mit einer Fixierstruktur 58, 58' ausgestattet, welche dazu dient, die Befestigung des proximalen Endes 59b durch Einführen der Befestigungsstruktur 58 in entsprechend geformte Haltestruktur zu erleichtern (Haltestruktur 33b in Figur a, nicht gezeigt in Figur 3a). Zwei mögliche Versionen der Befestigungsstruktur die hier gezeigt sind, sind eine scheibenförmige Befestigungsstruktur in Figur 3b sowie eine quaderförmige Befestigungsstruktur mit quadratischer Grundfläche in Figur 4b, doch sind dies nur zwei von vielen möglichen Varianten, welche im Rahmen vorliegender Erfindung realisiert werden könnten.

Der Wesentliche Vorteil der Ausführungsformen aus Figur 3 ist, dass der Instrumentenkanal 50b gerade entlang des gesamten Endoskops verläuft ohne irgendeinen scharfen Knick aufzuweisen, wodurch vorteilhafterweise die Reibung eines Instrumentendrahts der im Instrumentenkanal eingesetzt ist niedrig bleibt und hierdurch das Einsetzen sowie die Bewegung und präzise Kontrolle und Positionierung des Instruments während einer Operation erleichtert wird. Während in der Ausführungsform nach Figur 4 die Y-Abzweigung 56 einen etwas schärferen Knick, welcher die Reibung erhöhen wird, aufweist, hat sie den Vorteil, dass der Instrumentenkanal insgesamt kürzer ist, was die Erhöhung der Reibung durch den Knick teilweise rückgängig machen dürfte, und darüber hinaus das proximale Ende 59b an eine ergonomischere Position bringt, da Haltestruktur 3 während des Einfügens eines Instruments durch einen einzelnen Verwender des Endoskops welcher dieses mit einer Hand halten und mit der anderen Hand das Instrument führen muss, leichter zu erreichen ist, als dies bei Ausführungsform nach Figur 3 der Fall ist, bei der das proximale Ende des Instrumentenkanals mit dem proximalen Ende des Griffes 3 zusammenfällt.

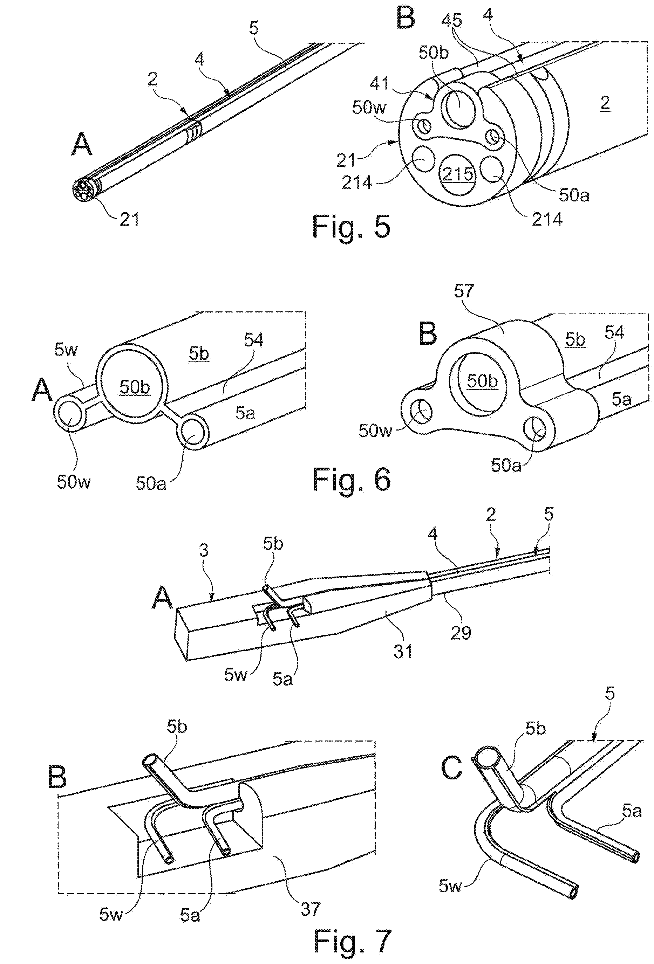

Die **Figuren 5** - **7** zeigen eine dritte bevorzugte Ausführungsform des Endoskops gemäß vorliegender Erfindung. Die **(Teil-)****Figur 5A** zeigt den Endoskopschaft 2 und die Figur 5b eine vergrößerte Ansicht seines distalen Endes 21.

Wie man am besten anhand **(Teil-)****Figur 5B** erkennen kann, ist die Kerbe 4 in dieser Ausführungsform weder u- noch omegaförmig wie in den anderen Ausführungsformen, sondern ist grob p-förmig und passt sich genau dem Querschnitt des Schlauches 5 an, welcher in dieser Ausführungsform aus 3 Teilschläuchen 5b', 5a' und 5w' besteht, welche jeweils einen Arbeitskanal bereitstellen. Der größte Teilschlauch, 5b', dient der Bereitstellung eines Instrumentenkanals 50b, die beiden kleineren Teilschläuche 5a' und 5w' der von Wasser- und Luftkanälen 50w respektive 50a. Die drei Teilschläuche 5b', 5a' und 5w' sind in einem spiegelsymmetrischen Dreieckmuster angeordnet und laufen parallel zu und beabstandet voneinander, wobei die Teilschläuche 5a' und 5w' mit dem kleineren Durchmesser mit dem größeren Teilschlauch 5b' durch dünne Brücken 54 aus dem gleichen Material wie die Schläuche verbunden sind.

Wie in Figur 5b gezeigt, ist das distale Ende des Schlauches 5 sicher in der Ausnehmung welche durch das distale Ende 41 der Kerbe 4 im distalen Ende 21 des Endoskopschafts 2 gebildet wird gehalten mit Hilfe einer durch eine Endkappe 57, welche über das distale Ende von Schlauch 5 vor der Installation des Schlauchs 5 in der Kerbe 4 geschoben wird. Die Kerbe 4 hat über ihre ganze Länge eine Breite welche ausreichend den Zugang zur Reinigung und Sterilisation des Endoskops bietet. Die Vorbereitung des Endoskops wird dadurch erleichtert, dass wie in den anderen beiden Ausführungsformen, auf scharfe innere Kanten verzichtet und auch die äußeren Kanten 45 der Kerbe 4 abgerundet sind. Letzteres hilft auch dabei, den mehrteiligen Schlauch 5 vor versehentlichem Zerschneiden oder andere Beschädigungen während des Installationsvorgangs zu beschützen.

Auch in dieser Ausführungsform hat der Endoskopschaft 2 einen kreisförmigen Querschnitt, abgesehen von der Kerbe 4, oder genauer gesagt ist die Komplettierung des Querschnitts, so wie oben definiert, ein Kreis. Dies ist allgemein die bevorzugte Form des Querschnitts von Endoskopschäften, da es die Querschnittsfläche im Verhältnis zum Umfang maximiert und somit die Unannehmlichkeit und Schmerz welches das Endoskop beim Einführen in den Patienten verursacht minimiert. Die Kerbe hat eine Tiefe, welche sich bis zum Flächenschwerpunkt des Querschnitts der Komplettierung des Schaftes erstreckt.

Aufgrund der Form des mehrteiligen Schlauches 5 kann seine Installation in der Kerbe 4 nur schwierig und unter größerer Deformation des Schlauches durch ein Anordnen des Schlauches durch ein Anordnen des Schlauchs oberhalb der Kerbe und anschließendes Eindrücken in den Sitz in der Kerbe durch Anwendung einer radialen Kraft, wobei es abschnittsweise von einem Ende des Schaftes bis zum anderen vorgegangen wird, wie es die schnellste und somit empfohlene Installationsmethode im Falle der anderen Ausführungsform von Figur 1 und 2 war. Im Gegensatz dazu wird der mehrteilige Schlauch 5 dieser Ausführungsform am besten in axialer Richtung in die Kerbe 4 beginnend am distalen Ende eingeschoben. Um dies zu erleichtern, ist die Größe des Querschnitts kleiner gewählt als der Querschnitt der Kerbe 4 um einen Freiraum zu lassen, da andernfalls die Reibung zwischen der Außenfläche des Schlauches 5 und den Wänden der Kerbe 4 eine einfache und schnelle Installation verhindern würde. Aufgrund dieses Freiraums ist ein sicherer Sitz des distalen Endes des Schlauches 5 in der Einbuchtung des distalen Endes des Schaftes 2 nicht länger garantiert und aus diesem Grund die Endkappe 58 bereitgestellt.

**Figur 7** zeigt, in Teilfiguren 7A und vergrößert in Teilfigur 7B das proximale Ende 29 des Endoskopschaftes 2, welches am distalen Ende 31 des Griffs 3 befestigt ist. Der Vorteil des dreiteiligen Layouts des Schlauches 5 wird bei der Betrachtung der Figuren 7A und 7B erkennbar: Da die Teilschläuche 5a' und 5w' leicht vom Schlauch 5b' an jeder beliebigen Stelle separierbar sind, ist es nicht länger nötig, eine Y-Abzweigung mit genau dem richtigen Winkel in genau dem richtigen Abstand vom distalen Ende des Schlauches 5 bereitzustellen. Die die Teilschläuche verbindenden Materialbrücken können im Gegenteil einfach an einem gewünschten Punkt getrennt und so die Teilschläuche voneinander gelöst und durch Auseinanderziehen voneinander separiert werden, beispielsweis innerhalb der von vorliegend dargestelltem Ausführungsbeispiel hierfür bereitgestellten Separationszone 37 in der Form einer Einbuchtung im Griff 3, und an dem entsprechenden Befestigungs- oder Anschlusspunkt befestigt bzw. angeschlossen werden: Der Instrumentenkanalschlauch 5b' wird am Griff befestigt, beispielsweise in Haltestrukturen wie in Figuren 3A und B bzw. 4A und B gezeigt, und der Wasser- und der (Druck-)Luftkanal 5w', 5a' werden an Ventile im Griff oder an sonstige Quellen kontrollierten Wasser- und Luftstroms angeschlossen.

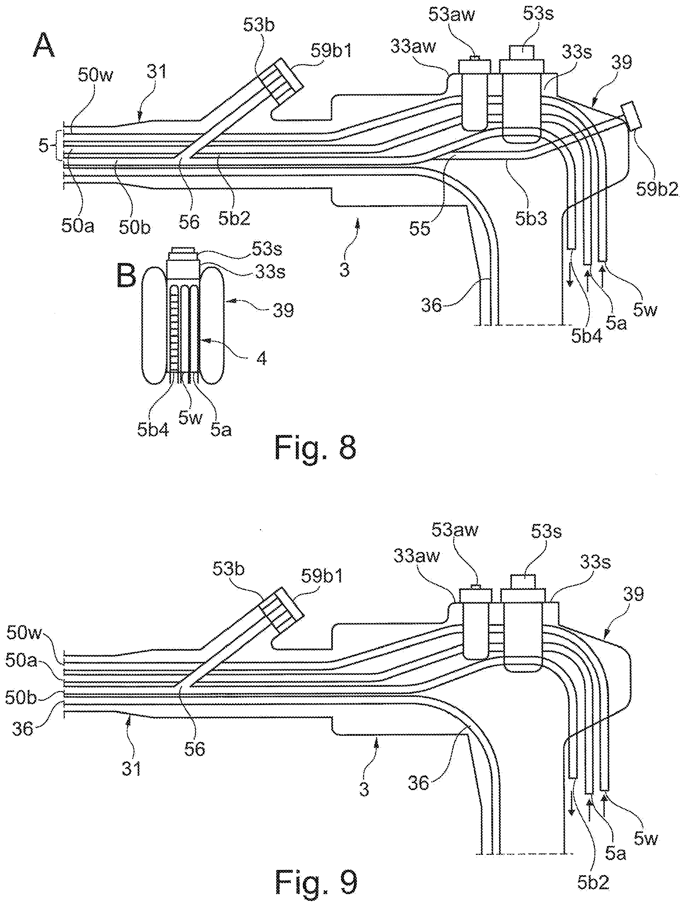

Figur 8 zeigt einen schematischen Längsschnitt durch den Griff einer weiteren Ausführungsform des erfindungsgemäßen Endoskops, welche die Elemente der Ausführungsformen aus den Figuren 3 und 4 kombiniert und dabei auch den genauen Verlauf der einzelnen Schläuche im Griff illustriert.

Die erfindungsgemäße, hier nicht dargestellte, Kerbe ist auch in dieser Ausführungsform im Griff 3 nahtlos fortgesetzt. Sie verläuft direkt oberhalb optischen Fasern bzw. elektrischen Kabel 36 welche durch den Griff 3 und den Schaft 2 zu den optischen Elementen (Linse, Lichtausgänge bzw. Kamerachip und LED) im distalen Ende des Schaftes führen. Der in der Kerbe eingesetzte Schlauch 5 umfasst einen kombinierten Instrumentier- und Absaugkanal 50b, einen Wasserkanal 50w und einen Luftkanal 50a. Der Instrumentierkanal 50b verzweigt sich in seinem Verlauf mehrfach: eine erste Y-förmige Abzweigung 56 ist im Bereich des distalen Endes 31 des Griffs 3 gelegen. Der abzweigende Ast 5b1 führt zu einem lösbar in einer ersten Haltestruktur 33b1 auf der Oberseite des Griffs fixierten ersten proximalen Ende 59b1, wobei dieser Ast in einem relative großen Winkel von ca. 30 Grad zum Verlauf des Schlauches 5 abzweigt. Der zweite Ast 5b2 des kombinierten Absaug- und Instrumentierkanals ist in der Kerbe weitergeführt bis zu einer Abzweigung 55, in welcher sich der Instrumentierkanal 50b erneut in einen dritten und einen vierten Ast aufspaltet wobei der dritte Ast 5b3 zu einem zweiten proximalen Ende 59b2 führt, welches in einer Halterung 33b2 im proximalen Ende 39 des Griffs 3 lösbar festgelegt ist. Der vierte Ast 5b4 des Instrumentierkanals wird zusammen mit dem Luftkanal 50a und dem Wasserkanal 50w zu Ventilen in Ventilblöcken 53aw, 53s geführt, welche in Halterungen 33aw und 33s in der Oberseite des Griffs 3 vorgesehen sind. Mittels des Ventils 53b lässt sich hierbei zum Zwecke der Absaugung kontrolliert ein Unterdruck in dem Instrumentierkanal 50b erzeugen, wozu das nicht dargestellte proximale Ende des vierten Asts 5b4 an eine Vakuumquelle angeschlossen werden muss. Gleichermaßen sind die vom Kombinationsventil 50aw zur Steuerung der Wasserspülung bzw. Luftinsufflation ausgehenden offenen Enden des Wasserkanals 50w und des Luftkanals 50a an entsprechende Quellen von Wasser und Druckluft anzuschließen. Wie im rechten Teil von (Teil-)Figur 8A zu sehen, werden Absaug-, Wasser und Luftkanal nach den Ventilen 53aw, 53b als unabhängige Teilschläuche 5b4, 5a und 5w in einer Verbreiterung der dort nach unten abbiegenden Kerbe 4 im proximalen Ende 39 des Griffes 3 nach unten abbiegend weitergeführt, wodurch sie die Bewegung des Endoskops und die Tätigkeit seines Verwenders möglichst wenig einschränken. Größe und Verlauf der Kerbe 4 im proximalen Griffende 39 sind auch in der schematischen Rückansicht des Endoskops in **(Teil-)****Figur 8B** dargestellt.

In **Figur 9** ist der Verlauf der Schläuche bzw. Kanäle im Griff der Ausführungsform des erfindungsgemäßen Endoskops nach Figur 4 detaillierter gezeigt. Er entspricht in großen Teilen dem der Ausführungsform aus Figur 8, mit dem Unterschied, dass im Instrumentierschlauch keine zweite Abzweigung vorhanden ist, und somit der zweite, in der Kerbe 4 weitergeführte Ast 5b2 des Instrumentierschlauches 5b nach der ersten Abzweigung 56 zusammen mit den Versorgungsschläuchen für Wasser 5w und (Druck-)Luft 5a direkt zu den Ventilblöcken in den Halterungen auf der Oberseite des Griffs 3 geführt ist, genauer im Falle des zweiten Astes des Instrumentierschlauches 5b2 zum Absaugventil 53s.

Die Rückansicht des Endoskopgriffs aus Figur 8B ist auch auf die Ausführungsform der Figur 9 übertragbar.

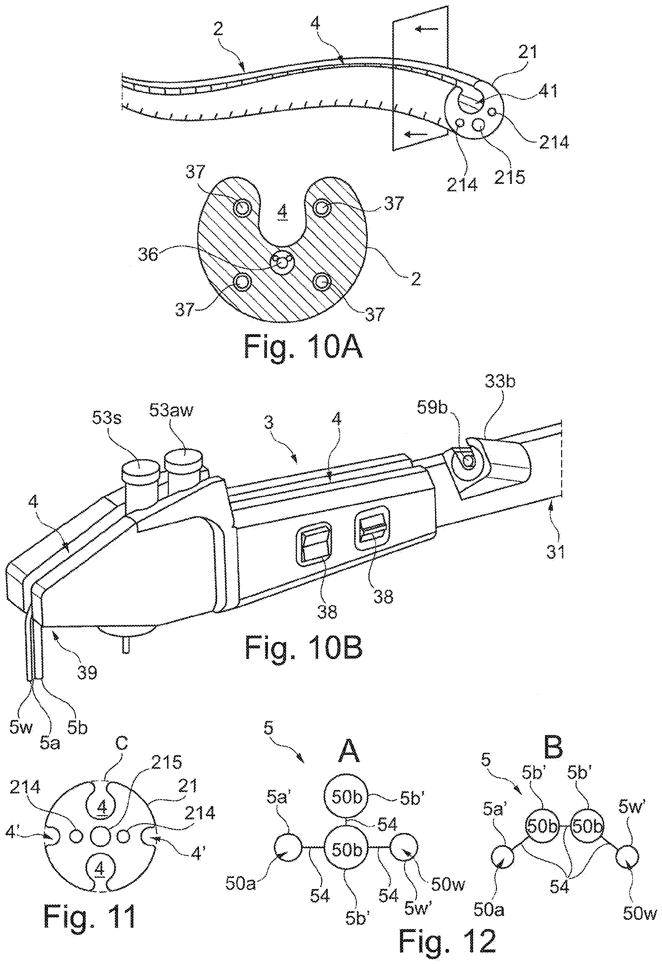

**Figur 10** zeigt in zwei Teilfiguren 10A und 10B eine Ausführungsform des erfindungsgemäßen Endoskops mit einem Vollmaterialschaft.

Der obere Teil von (Teil-)**Figur 10A** zeigt den Schaft 2 mit der für vorliegende Erfindung charakteristischen tiefen Kerbe 4 auf der Außenseite, welche im Distalen Ende 21 des Schaftes 2, welches auch das optische Beobachtungsmittel 215 und die Beleuchtungsmittel 214 beherbergt, in der Aussparung 41 mündet.

Der untere Teil von Figur 10A zeigt einen Querschnitt in oben angedeuteter Schnittfläche. Der Schaft 2 ist, wie dargestellt aus einem Vollmaterial gefertigt, in welches die Hydraulikleitungen 37 und ein Kanal mit Steuerleitungen 36 zur Ansteuerung der optischen bzw. Beleuchtungskomponenten 214, 215 eingebettet sind. Das Vollmaterial ist bevorzugt ausreichend Steif um eine gute Positionierbarkeit der Endoskopspitze 21 auch gegen den Widerstand des Gewebes des Patienten zur ermöglichen, aber gleichzeitig flexibel genug, um Gebeschäden auszuschließen.

(Teil-)**Figur 10B** zeigt eine Ausführungsform eines Griffes 3 eines Endoskops mit Vollmaterialschaft und Hydrauliksteuerung wie in Figur 10A. Die über die ganze Länge des Griffes 3 fortgesetzte Kerbe 4, Haltestruktur 33b nahe dem distalen Griffende 31, Absaugventilblock 53s, kombinierte Wasser- und Luftventilblock 53aw, Instrumentieröffnung 59b und Teilschläuche 5b, 5a und 5w sind aus den Ausführungsformen in Figuren 4 bzw. 9 bekannt. Der Unterschied vorliegender Ausführungsform besteht darin, dass anstelle von Kontrollrädern Kipp bzw. Wippschalter 38 zur Steuerung der Biegung des Schaftes 2 eingesetzt sind. Über die Wippschalter 38 kann der Bediener des Endoskops den auf die Hydraulikleitungen 37 beaufschlagten Druck steuern. Eine Biegung des Schaftes 2 resultiert aus einer Druckdifferenz zwischen den Hydraulikleitungen 37.

**Figur 11** zeigt eine Vorderansicht der Endoskopspitze einer Ausführungsform des erfindungsgemäßen Endoskops, bei welcher der Schaft über zwei Kerben zur Aufnahme eines Instrumentenschlauchs verfügt. Ähnlich den Ausführungsformen aus den Figuren 1 und 2 verfügt das distale Ende 21 des Endoskopschaftes über eine zentral angeordnete Optik 215 mit zwei seitlich angeordneten Lichtausgängen 214. In einer Linie mit den Lichtausgängen 214 sind zwei U-förmige Kerben 4' kleineren Durchmessers zur Aufnahme je eines Wasser- und (Druckluftschlauches) vorhanden. Spiegelsymmetrisch oberhalb und unterhalb der durch die Kerben 4' definierten Ebene sind Ω-förmige Kerben 4 angeordnet, welche jeweils ausreichend groß sind, um Instrumentenschläuche aufnehmen zu können. Hierdurch können bei einem Endoskop gemäß dieser Ausführungsform zwei Instrumente zugleich eingesetzt werden. Es ist also ein beidhändiges Arbeiten ermöglicht, was bei manchen Untersuchungen oder Operationen eine Vereinfachung oder Beschleunigung bedeuten kann. Um trotz der beiden vergleichsweise tiefen Kerben 4 noch ausreichend Raum für die Ansteuerung der Optik 215 und der Lichtausgänge 214 sowie der die Beweglichkeit des Schaftes ermöglichenden Bowdenzüge oder Hydraulikleitungen zu haben, weißt der Schaft in dieser Ausführungsform einen leicht elliptischen Umriss bzw. eine leicht elliptische Komplettierung C auf, wobei die große Halbachse in Richtung der tiefen Kerben weist.

Ein Ähnliches Ziel verfolgen die in **Figur 12** dargestellten Ausführungsformen eines aus mehreren Teilschläuchen bestehenden Schlauches. Der Schlauch 5 besteht in beiden Varianten nach Figur 12A und Figur 12B aus vier Teilschläuchen, nämlich zwei kleineren Versorgungsschläuchen 5a', 5w' und zwei größeren Arbeitsschläuchen 5b'.

In der (Teil-)**Figur 12A** sind die Teilschläuche etwa in Form eines umgedrehten "T" angeordnet, wobei die beiden Arbeits- bzw. Instrumentenschläuche 5b', welche Kanäle 50b für Instrumente oder zur Absaugung bereitstellen, mittig übereinander verlaufen und über eine dünne Brücke 54 miteinander verbunden sind. Die kleineren Schläuche 5a' und 5w' mit einem Luftkanal 50a bzw. Wasserkanal 50w verlaufen seitlich des unteren Teilschlauchs 5b' und sind an diesen über Materialbrücken 54 angeschlossen.

In der (Teil-)**Figur 12B** sind die beiden Arbeits- bzw. Instrumentenschläuche 5b', welche Kanäle 50b für Instrumente oder zur Absaugung bereitstellen, nebeneinander angeordnet. An beide schließt sich seitlich nach unten versetzt und über Brücken 54 verbunden je ein kleinerer Versorgungschlauch an: links ein Luftschlauch 5a' mit dem Luftkanal 50a und rechts der Wasserschlauch 5w' mit dem Wasserkanal 50w.

Beide Ausführungsformen des Schlauches erlauben bei Verwendung in einem Endoskop mit einer komlementär geformten Kerbe den gleichzeitigen Einsatz von zwei Instrumenten.

Bei den Ausführungsformen des erfindungsgemäßen Endoskops mit doppeltem Instrumentenschlauch sind entsprechend auch zwei proximale Zugänge nötig, um die Instrumente unabhängig voneinander in die jeweiligen Kanäle einschieben zu können. Diese Zugänge können entweder beide im distalen Bereich des Griffes oder beide am proximalen Ende des Griffes vorgesehen und in entsprechenden Haltestrukturen fixiert werden. Alternativ kann auch wie in Figur 8 dargestellt, ein Zugang distal am Griff und das andere proximal am Griff festgelegt sein, d.h. die Ausführungsformen des Endoskopschaftes nach Figur 11 und des Schlauchs zusammen mit einem Schaft mit einer komplementär geformten Kerbe sind vorteilhaft mit der Ausführungsform des Griffes nach Figur 8 kombinierbar.

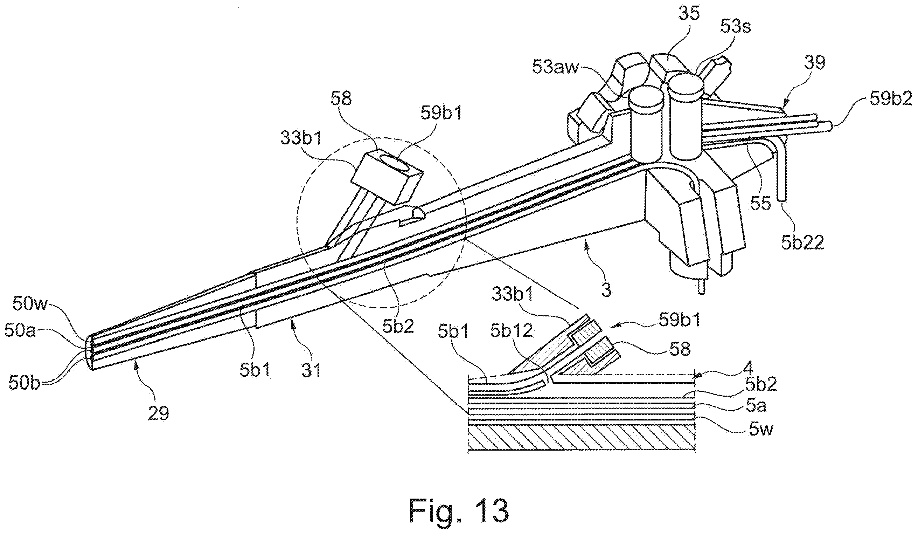

Auch bei vorliegender Ausführungsform mit doppelläufigem Instrumentierkanal ist es grundsätzlich ausreichend, weiterhin nur ein einzelnes Absaugventil, also die Verwendung nur eines der Instrumentierkanäle als Absaugkanal vorzusehen. Um jedoch zu verhindern, dass der nicht zur Absaugung eingerichtete Kanal, dies ist üblicherweise derjenige, dessen Zugangsöffnung distal im Griff fixiert ist, sich bei wiederholtem durchfahren mit einem Instrument, etwa einer Biopsiezange, mit Verunreinigungen zusetzt, welche bei einer Drucksteigerung am Operationsort aus der Zugangsöffnung hervorquellen und die Außenseite des Endoskops kontaminieren könnten, wird vorgeschlagen, beide Instrumentierkanäle über eine offene, kurzstreckige Verbindung in fluide Kommunikation miteinander zu setzen. Bevorzugt wird diese Verbindung im Bereich des Griffes, soweit proximal, als möglich, vorgesehen. Durch das Herstellen einer solchen offenen Verbindung wird erreicht, dass auch der zweite Biopsiekanal sozusagen automatisch mit abgesaugt und so von Verunreinigungen freigehalten wird. Eine Variante des erfindungsgemäßen Endoskops mit doppelläufigem Instrumentenkanal ist in **Figur 13** dargestellt.

Die Abbildung zeigt den Griff 3 mit der oberseitigen Kerbe 4 in dem der wechselbare Schlauch 5 bestehend aus vier Teilschläuchen einem ersten Instrumentenschlauch 5b1, einem zweiten Instrumentenschlauch 5b2, einem Druckluftschlauch 5a und einem Wasserschlauch 5w, verläuft. Das proximale Ende des ersten Instrumentenschlauchs 5b1 mit der Zugangsöffnung 59b1 ist in der Halterung 33b1 im distalen Bereich 31 des Griffs 3 fixiert. Der auch der Absaugung dienende zweite Instrumentenschlauch 5b2 ist in der Kerbe 4 über das Absaugventil 53s bis zum proximalen Griffende geführt, wo die Zugangsöffnung 59b2 zum zweiten Instrumentenschlauch fixiert ist. Ein Ast 5b22 des zweiten Instrumentenschlauches setzt sich von der Abzweigung 55 bis zu einem Anschluss an eine Vakuumquelle fort. Wasser- und Luftschlauch 5w bzw. 5a verlaufen über Kombinationsventil 53aw bis zum proximalen Ende 39 des Griffs 3, wo sie die Kerbe 4 und das Endoskop 1 verlassen und sich zu den jeweiligen Anschlüssen fortsetzen.

Die untere Teilzeichnung zeigt einen vergrößerten Ausschnitt des distalen Griffendes 31 mit Haltestruktur 33b1 oberhalb der Kerbe 4 zur Fixierung der Fixierstruktur 58 des proximalen Endes des ersten Instrumentierkanals 5b1 mit Zugangsöffnung 59b1. Eine kurze offene Verbindung 5s12 stellt eine fluide Kommunikation zwischen beiden Instrumentierkanälen 5b1 und 5b2 sicher, so dass auch der nicht unmittelbar an das Absaugventil angeschlossene erste Instrumentierkanal 5b1 mit abgesaugt wird und von Verunreinigungen frei bleibt.

Gezeigt ist ein zwar mit Kontrollrädern 35 ausgestattetes Endoskop, jedoch ist die Art der Steuerung von der Ausgestaltung der Kerbe und der Schläuche weitgehend unabhängig und auch mit, beispielsweise, einer Hydrauliksteuerung kompatibel.

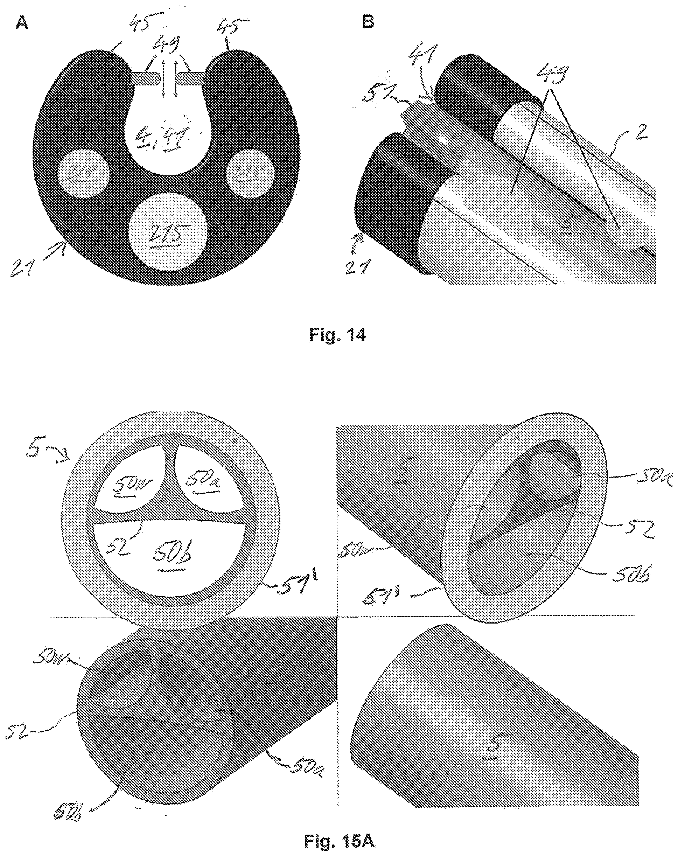

In der **Figur 14** sind in zwei Teilfiguren zwei Ansichten einer Ausführungsform des erfindungsgemäßen Endoskops dargestellt, in welcher der Schlauch zumindest im Bereich des distalen Schaftendes mittels elastischer Halteflügel in der Kerbe gehalten wird.

Teilfigur A zeigt eine Vorderansicht ohne Schlauch, Teilfigur B hingegen eine überhöhte perspektivische Ansicht mit in die Kerbe 4 eingesetztem Schlauch 5.

Die Halteflügel 49 ragen in den Hals der □-förmigen Kerbe 4 hinein. Wie durch die Pfeile angedeutet sind die Halteflügel 49 elastisch, etwa aus Gummi, und können so beim Einsetzen und Herausnehmen des Schlauches in die bzw. aus der Kerbe 4 nachgeben. Wie in Teilfigur B zu erkennen, sind die Halteflügel 49 axial versetzt angeordnet. Der vorderste Halteflügel 49 ist kurz hinter der das distale Ende des Schaftes 2 bildenden Kappe angesetzt.

Das distale Ende des Schlauchs 5 ist durch die Fixiernase 51 an einer Rotation relativ zur Kerbe gehindert, vornehmlich um die relative Ausrichtung des Wasserkanals zur Optik 215 und der Beleuchtung 214 zu fixieren.

Die oben Teilfiguren von **Figur 15A** zeigen zwei Ansichten einer Ausführungsform des erfindungsgemäßen flexiblen Schlauches, welche eine wulstartige, ringförmige Verdickung aufweist, welche eine Rückgleitsicherung bildet gegen Zurückgleiten des Schaftendes bei einer Längenänderung der Kerbe im Endoskopschaft bei Verbiegung des Schaftes. Die unteren Teilfiguren zeigen im Vergleich hierzu einen Schlauch ohne Rückgleitsicherung

Die kreisringartige Verdickung 51' ist integral mit dem distalen Ende des Schlauches 5, und besteht aus demselben Material wie der Rest des Schlauches inklusive der Unterteilungswände 52, welche in bereits oben beschriebener Weise das Schlauchinnere in drei parallele Kanäle, einen Arbeitskanal 50b, einen Wasserkanal 50w und einen Luftinsufflationskanal 50a unterteilen.

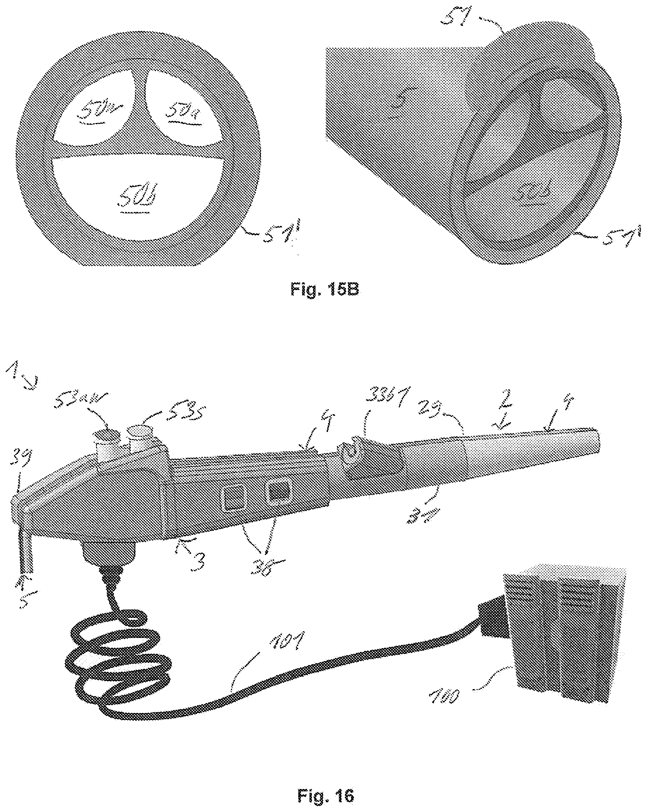

In **Figur 15B** ist eine Ausführungsform des distalen Schlauchendes dargestellt, bei welcher die Rückgleitsicherung durch eine kreisringförmige Kunststoffscheibe gebildet ist, welche am distalen Ende des Schlauches 5 befestigt, etwa angeklebt oder -geschweißt, ist. Zusätzlich ist auch eine Erhebung 52 aus demselben Kunststoffmaterial in die Scheibe 51' integriert, welche als Fixiernase 52 dient.

Die **Figur 16** zeigt eine weitere perspektivische Ansicht der hydraulisch betätigten Ausführungsform des erfindungsgemäßen Endoskops aus Figur 10B. Das Endoskop 1 verfügt über einen Schaft 2, welcher über ein proximale Schaftende 29 in das distale Ende 31 des Griffteils 3 übergeht. Im Bereich des distalen Griffteilendes 31 ist eine Fixierstruktur 33b1 für einen Biopsiezugang angeformt. Die Kerbe 4, in welcher erfindungsgemäß der mehrere Arbeitskanäle umfassende Schlauch 5 eingesetzt ist, wobei mindestens ein freier Abschnitt vorhanden ist, in welchem der Schlauch in der Kerbe Spiel hat, so dass er sich relativ zu den Kerbenwandungen leicht axial verschieben, aber auch tangential verdrehen kann, zieht sich vom (nicht sichtbaren) distalen Schaftende auf der Oberseite durch den kompletten Schaft 2, setzt sich im Griffteil 3 fort und führt dort bis zum proximalen Griffende 39.Absaug- Wasser- und Luft(teil)schläuche sind über eine Kerbe im Griffende 39 nach unten geführt.

In den Schlauch 5 sind integrale Ventilblöcke 53aw, mit einem Luft- und einem Wasserventil und 53 mit einem Absaugventil eingearbeitet, welche in komplementäre Halterungen auf der Oberseite des Endoskops eingesetzt sind.

Zur Steuerung der Verbiegung des Schaftes 2 sind an der Seite des Griffstücks zwei Wippschalter 38 angeordnet, mit denen ein Benutzer die Verbiegung des Schaftes 2 in Links-Rechts Richtung, der andere in der dazu senkrechten Oben-Unten Richtung steuern kann. Die im Griffstück 3 integrierte Hydraulik wird über das Verbindungskabel 101 vom Prozessor 100 mit elektrischer Energie versorgt.

Der große Vorteil dieser Ausführungsform ist ihre leichte Handhabbarkeit. Bisher war es üblich, Endoskope mit einem Versorgungsstrang zu versehen, in welchem die vergleichsweise steifen, aus dem Endoskopschaft heraustretenden Wasser-, Luft und Absaugkanäle zusammengefasst waren. Diese waren nicht wie hier als herausnehmbare, disposable Schläuche ausgestaltet, sondern dauerhaft mit dem Gerät verbunden. Der steife Versorgungsstrang machte den Einsatz von üblichen Endoskopen sehr unhandlich. Da vorliegende lediglich ein flexibles Verbindungskabel 101 an die Energieversorgungseinheit 100 angeschlossen ist, und die separat am proximalen Griffende aus dem Endoskop 1 zu den jeweiligen Versorgungseinheiten geführten Schläuche 5 flexibel sind.

Da die Wippschalter 38 hier die üblichen Drehräder ersetzen, ist auch eine Flächendesinfektion des Endoskopgriffs deutlich vereinfacht.

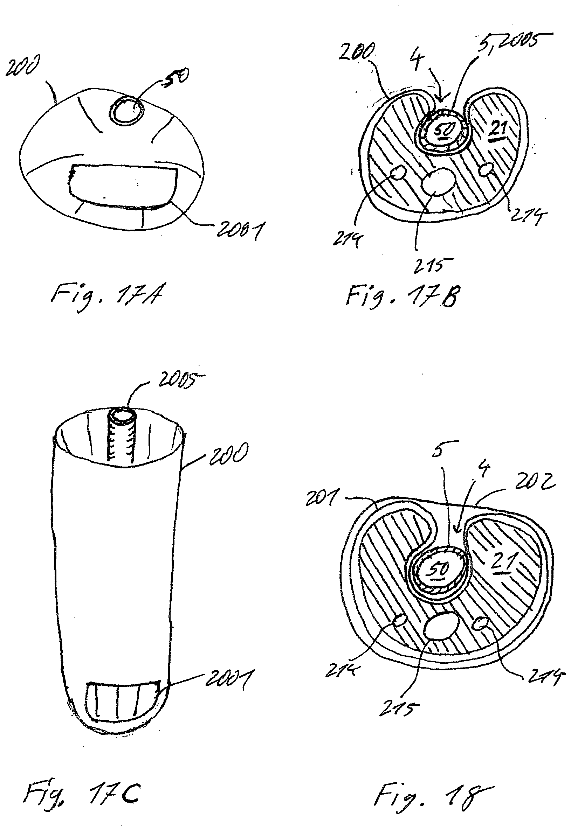

Die **Figuren 17A** - **C** stellen eine weitere bevorzugte Ausführungsform des erfindungsgemäßen Endoskops dar, bei der der in die Kerbe eingesetzte Schlauch in eine den Endoskopschaft eng anliegend umschließende, aus einem hochelastischen Material bestehende Kontaminationsschutzhülle integriert ist.

Die **Figuren 17A** und **17B** zeigen die Schutzhülle 200 ohne Endoskopschaft. In Figur 17A ist dabei eine Vorderansicht und in Figur 17B eine aufgeschnittene perspektivische Ansicht der Schutzhülle 200 gezeigt. Wie dargestellt mündet der integrierte Schlauch 205 in einer Mündung 50 im oberen Bereich. Im einem unteren Bereich ist ein transparentes Fenster 2001 vorgesehen, welches, nachdem die Hülle auf den Endoskopschaft aufgezogen und dabei der integrierte Schlauch 205 in die Kerbe 4 im Endoskopschaft eingeklickt wurde, mit der Optik in Form der Linse 215 und der Beleuchtungen 2014 in der Spitze 21 des Schaftes 2 zusammenfällt, so dass die Verwendung der Optik durch die Hülle 200 nicht behindert wird. Statt der gezeigten Form kann das Fenster 2001 auch größer ausgeführt werden. Beispielsweise kann die gesamte Spitze der Hülle 200 transparent ausgeführt sein.

In den Figuren ist vereinfachend ein einlumiger Schlauch dargestellt, jedoch kann in alternativen Ausführungen auch ein mehrlumiger Schlauch, entweder ein Schlauch mit in etwa rundem oder elliptischem Querschnitt und internen Unterteilungswänden oder ein Schlauch mit über dünnen Brücken verbundenen runden oder elliptischen Teilschläuchen, verwendet werden.

Die Figur 17B dargestellte Form der Hülle 200 entspricht der Form, welche sie durch das Aufziehen auf den Schaft 2 erhält und nicht der Form vor dem Aufziehen. In letzterer ist die Hülle 200 bevorzugt platzsparend zu ihrer kappenartigen Spitze hin zu einem Torus aufgerollt. Zum Aufsetzen auf den Schaft würde zunächst die Kappe auf die Schaftspitze 21 aufgeschoben und die Hülle 200 dann kondomartig über den Schaft abgerollt, wobei der in die Hülle 200 integrierte Schlauch 205 in die Kerbe 4 im Schaft 2 eingedrückt oder -gepresst wird.

Der dann erreichte Zustand ist in der **Figur 17C** illustriert, welche einen Querschnitt durch die Schaftspitze 21 mit aufgesetzter Hülle 200 zeigt.

In **Figur 18** ist ähnlich zu Figur 17C ein Querschnitt durch die Schaftspitze eines erfindungsgemäßen Endoskops dargestellt, jedoch sind hier zwei Hüllen verwendet, welche außerhalb der Kerbe 4 beide durch ihre inhärente Elastizität eng an die Mantelfläche des Schaftes 2 anliegen. Für die innere Hülle 201 gilt dies jedoch auch innerhalb der Kerbe 4, wobei sie hier durch den separaten in die Kerbe 4 eingesetzten Schlauch 5 an die Innenwandungen der Kerbe 4, mehr oder weniger stark angepresst wird. In den erfindungsgemäßen freien Bereichen des Schlauches, in welchen dieser sich relativ zur Kerbe leicht bewegen kann ist der Anpressdruck geringer bis nicht vorhanden, in den Bereichen des Schlauches außerhalb der freien Bereiche, in denen der Schlauch 5 fest in der Kerbe 4 gehalten ist, höher. Die äußere Hülle 202 umschließt innere Hülle 201 und den Schlauch. Da sie nicht durch den Schlauch 5 in die Kerbe gedrückt wird, entspricht der sich durch die Elastizität der Hülle 202 ausbildende Querschnitt nahezu der mathematischen konvexen Hülle des Schaftquerschnittes.

### Bezugszeichenliste

- 1: Endoskop
- 2: Schaft
- 21: Distales Ende von Schaft 2
- 214: Lichtausgang
- 215: Optische Linse
- 29: Proximales Ende des Schaftes 2
- 3: Griff
- 31: Distales Ende von Griff 3
- 33b: Haltestruktur für Befestigungsstruktur
- 33b1: erste (distale) Haltestruktur für Fixierstruktur 58
- 33b2: zweite (proximale) Haltestruktur für Fixierstruktur 58'
- 33a, 33w: Haltestruktur für Ventilblöcke
- 35: Kontrollräder
- 36: optische Fasern/elektrische Kabel
- 37: Hydraulikleitung
- 38: Wippschalter
- 39: Proximales Ende von Griff 3
- 4: Hauptkerbe
- 4': Zusätzliche Kerben
- 41, 41': Distales Ende der Kerben 4, 4'
- 45, 45': Äußere Kanten der Kerben 4, 4'
- 49: Halteflügel
- 5: Arbeitskanalschlauch
- 5b, 5b': Instrumentierkanalschlauch
- 5b1: Erster (Ast des) Instrumentierkanalschlauch(s)
- 5b2: Zweiter (Ast des) Instrumentierkanalschlauch(s)
- 5b22: Ast des zweiten Instrumentierkanalschlauchs
- 5b3: Dritter Ast des Instrumentierkanalschlauchs
- 5b4: Vierter Ast des Instrumentierkanalschlauchs
- 5a, 5a': Luft- / Unterdruckkanalschlauch
- 5w, 5w': Wasserkanalschlauch
- 5s12: offene Verbindung
- 50: Kanal im Inneren von 5
- 50b: Instrumentenkanal (Auslass)
- 50a: Luft- / Unterdruckkanal (Auslass)
- 50w: Wasserkanal (Auslass)
- 51: Fixiernase
- 51: Rückgleitsicherungsscheibe
- 52: Unterteilungswände
- 53b, 53b': Fixierstruktur,
- 53a, 53w: Ventilblöcke, Luft-/Unterdruck und Wasser
- 53s: Ventilblock Absaugung
- 53aw: Kombinationsventilblock Luft und Wasser
- 54: Brücken zur Verbindung der Teilschläuche
- 55: Y-Abzweigung mit kleinem Winkel
- 56: Y-Abzweigung mit größerem Winkel
- 57: Endkappe eines mehrteiligen Schlauchs
- 58, 58': Fixierstruktur
- 59b: Proximales Ende des Instrumentierkanals
- 59b1: Erstes Proximales Ende des Instrumentierkanals
- 59b2: zweites Proximales Ende des Instrumentierkanals
- 100: Prozessor
- 101: Verbindungskabel
- 200: Hülle
- 2001: Fenster in 200
- 2005: in 200 integrierter Schlauch
- 201: innere Hülle
- 202: äußere Hülle

- C: Komplettierung des Schaftquerschnitts
- CH: Konvexe Hülle des Schaftquerschnitts
- CoA: Flächenschwerpunkt der Komplettierung

## Patentansprüche

1. Endoskop (1) für die minimalinvasive Untersuchung oder chirurgische Behandlung eines Patienten, umfassend
- einen flexiblen, schlauchartigen Schaft (2) zum Einführen in den Patienten, mit einem distalen Ende (21), und
- einem Griff (3) um das Endoskop (1) zu halten mit einem distalen Ende (31), welches an einem proximalen Ende (29) des Schaftes (2) befestigt ist,
- wobei das Endoskop (1) mindestens einen Kanal (50b, 50w, 50a) zum internen Führen eines chirurgischen Instruments und/oder von Wasser und/oder Luft oder zur Verfügungsstellung eines Unterdrucks am distalen Ende (21) des Schaftes (2) aufweist,
- mindestens eine Kerbe (4, 4') sich in im wesentlichen axialer Richtung entlang der Außenseite des Schaftes (2) von einem Bereich in der Nähe des proximalen Endes (29) bis zum distalen Ende (21) erstreckt, wo die Kerbe (4, 4') in einer Einbuchtung (41, 41') im distalen Ende (21) mündet, und
- der zumindest eine Kanal (50b, 50w, 50a) durch zumindest einen hohlen Schlauch (5, 5b, 5a, 5w), welcher lösbar in die Kerbe (4, 4') eingesetzt und befestigt ist, bereitgestellt ist, und
- wobei eine konvexe Hülle (CH) des Schaftes (2) oder eine Komplettierung (C) des Schaftes (2) zu einer einfachen geometrischen Form ohne den hohlen Schlauch (5, 5b, 5a, 5w) der konvexen Hülle oder der Komplettierung (C) des Schaftes (2) mit dem hohlen Schlauch (5, 5b, 5a, 5w) entspricht.
**dadurch gekennzeichnet, dass**
der Schlauch (5, 5b, 5a, 5w) zur besseren Anpassung an Längenänderungen der Kerbe in Folge von Verbiegungen des Endoskopschaftes mindestens einen freien Abschnitt aufweist, in dem Kerbe (4, 4') und Schlauch (5, 5b, 5a, 5w) relativ zueinander derart dimensioniert sind, dass der Schlauch (5, 5b, 5a, 5w) dort in axialer Richtung, aber auch hinsichtlich einer Drehung des Schlauches um die lokale Längsachse der Kerbe, Spiel hat, und hierzu im gerade ausgestreckten Zustand des Endoskopschaftes (2) einen Außenquerschnitt aufweist, der etwas kleiner ist, als ein Innenquerschnitt der Kerbe (4, 4'), und
jenseits des freien Abschnitts bzw. zwischen und/oder jenseits der freien Abschnitte liegende Abschnitte des Schlauchs (5, 5b, 5a, 5w) vorhanden sind, welche fester in die Kerbe (4, 4') eingesetzt, und zwar entweder die Elastizität des Schlauches ausnutzend eingepresst oder alternativ oder zusätzlich mit einem nicht sehr klebekräftigen Klebstoff eingeklebt, sind.

2. Endoskop nach Anspruch 1, **dadurch gekennzeichnet, dass** der Schlauch (5, 5a, 5b, 5w) in dem freien Abschnitt eine höhere Verformbarkeit aufweist indem er
- faltenbalg- und/oder spiralschlauchartig ausgestaltet ist, und/oder
- aus einem Material höherer Dehnbarkeit besteht, und/oder
- dünnwandiger ist.

3. Endoskop nach Anspruch 2, **dadurch gekennzeichnet, dass** der Schlauch (5, 5a, 5b, 5w) zwei oder mehr, insbesondere drei oder mehr, freie Abschnitte mit höherer Verformbarkeit aufweist, welche durch normale Schlauchabschnitte verbunden sind, wobei diese, insbesondere alle im Wesentlichen gleich langen, normalen Abschnitte mittels komplementärer Dimensionierung oder eines leicht lösbaren Klebstoffes fest in der Kerbe (4, 4') gehalten sind.

4. Endoskop nach Anspruch 1 oder 2, **dadurch gekennzeichnet, dass** der Schlauch (5, 5a, 5b, 5w) über die ganze Länge des Schaftes (2), insbesondere die ganze Länge der Kerbe (4, 4') mit Spiel (4, 4') in die Kerbe eingesetzt ist, insbesondere einen Außendurchmesser aufweist, der zumindest im gerade gestreckten Zustand des Schaftes (2) kleiner ist, als ein Innendurchmesser der Kerbe (4, 4'), und
- mittels zweier oder mehr, insbesondere dreier oder mehr in die Kerbe (4, 4') ragender elastischer Halteflügel (49) gegen ein Herausgleiten aus der Kerbe (4, 4'), und/oder
- durch eine Rückgleitsicherung, insbesondere eine Ringscheibe (52), an einem distalen Ende des Schlauches (5, 5a, 5b, 5w), welches in das distale Ende der Kerbe (41, 41') eingesetzt ist, an einem Zurückgleiten in die Kerbe (4, 4') bei Verbiegung des Schaftes (2) gesichert ist.

5. Endoskop nach einem der Ansprüche 1 - 4, **dadurch gekennzeichnet, dass** die Kerbe (4, 4')
- abgerundete Ecken (45) hat, und/oder
- über die gesamte Länge des Schaftes (2) vom distalen Ende (21) bis zum proximalen Ende (29) erstreckt und insbesondere weiterhin kontinuierlich, bevorzugt in einer Oberseite des Griffes (3) vorgesetzt ist, und/oder
- einen Querschnitt hat, der U- oder Ω-förmig und konstant entlang des Schaftes ist und/oder
- spiralförmig um den Schaft (2) herum läuft, wobei der spiralförmige Verlauf einen Winkel von zwischen 0 und 180°, bevorzugt zwischen 30 und 150°, abdeckt, und/oder
- einen Querschnitt hat, der U- und/oder Ω-förmig und entlang des Schaftes nicht konstant ist, sondern insbesondere variiert
∘ zwischen einer U- und einer Ω Form, und/oder
∘ in seiner Querschnittsfläche, wobei insbesondere eine minimale Querschnittsfläche und/oder eine Ω-Form der Kerbe (4) am oder in der Nähe des distalen Endes (21) erreicht ist, und/oder
∘ am proximalen Schaftende (29) und/oder im Griff (3) einen vergrößerten Querschnitt hat zum Einsetzen in den Schlauch (5) integrierter Ventile in Ventilhalterungen (33b, 33a, 33w) des Griffes (3).

6. Endoskop nach einem der Ansprüche 1-5, **dadurch gekennzeichnet, dass** der Griff (3) zumindest eine Haltevorrichtung (33b, 33a, 33w)
- zum Einstecken eines Ventils eines Wasserkanals (50w) oder eines Ventils eines Luft-/Unterdruckkanals (50a), insbesondere eine oder mehrere Aussparungen (33a, 33w) im Griff (3), wobei insbesondere die Ventile in Ventilblöcken (53w, 53a), die einstückig mit dem Schlauch verbunden sind, eingelassen sind, um ein Einstecken in die Haltevorrichtungen (33w, 33a) zu erleichtern, und/oder
- zur Befestigung des proximalen Endes (59b) eines Instrumentierkanals (50b) des Schlauchs (5, 5b), wobei das proximale Ende (59b) bevorzugt eine Fixierungsstruktur (58, 58') zur Erleichterung des Einsteckens in eine Haltevorrichtung aufweist.

7. Endoskop nach einem Ansprüche 1 - 6, **dadurch gekennzeichnet, dass** der Schaft (2)
- einen im Wesentlichen symmetrischen Querschnitt aufweist, bevorzugt einen kreisförmigen, elliptischen oder ovalen Querschnitt und die Kerbe (4, 4') in einer Symmetrieebene des Schaftes (2) liegt,
- aus Vollmaterial, insbesondere einem Kunststoff, gefertigt ist, und/oder
- zwei zur Aufnahme eines Instrumentierschlauches (5b) geeignete Kerben (4) aufweist.

8. Endoskop gemäß einem der Ansprüche 1 - 7, **dadurch gekennzeichnet, dass** die Kerbe (4, 4'), bevorzugt entlang ihrer gesamten Länge, einen Flächenschwerpunkt (CoA) einer konvexen Hülle (CH) oder einer Komplettierung (C) eines Querschnitts des Schaftes (2) enthält.

9. Endoskop nach einem der Ansprüche 1 - 8, **dadurch gekennzeichnet, dass** der Schlauch (5, 205)
- einen Querschnitt hat, der komplementär zu einem Querschnitt der Kerbe (4, 4') ist, und
- aus einem elastischen Material besteht und einen Durchmesser aufweist, der zumindest an zwei Punkten entlang des Schaftes (2) größer als ein Durchmesser der Kerbe (4, 4') ist, wobei zumindest einer dieser mindestens zwei Punkten in der Nähe des distalen Endes (21) des Schaftes (2) liegt, wodurch der Schlauch (5) nach dem Einstecken in die Kerbe (4, 4') aufgrund elastischer Spannkräfte gehalten ist, und/oder
- in der Kerbe (4, 4') durch den nicht sehr klebekräftigen Klebstoff befestigtist.

## Claims

1. Endoscope (1) for the minimally invasive examination or surgical treatment of a patient, comprising
- a flexible tubular shaft (2) for insertion into the patient, with a distal end (21), and
- a grip (3) for holding the endoscope (1), a distal end (31) of the grip (3) being attached to a proximal end (29) of the shaft (2),
- wherein the endoscope (1) is provided with at least one working channel (50b, 50w, 50a) for internally guiding a surgical instrument and/or carrying water or air to or providing suction at the distal end (21) of the shaft (2),
- at least one groove (4, 4') extending substantially axially along the outer face of the shaft (2) from the vicinity of the proximal end (29) to the distal end (21), where the groove (4, 4') ends in a recess (41, 41') in the distal end (21), and
- the at least one working channel (50b, 50w, 50a) is provided by at least one hollow tube (5, 5b, 5a, 5w) that is removably housed in the groove (4, 4'), and
- wherein a convex hull (CH) or a completion (C) of the shaft (2) to a simple geometric shape without the hollow tube (5, 5b, 5a, 5w) is the same as a convex hull (CH) or completion (C) of the shaft with the hollow tube (5, 5b, 5a, 5w),
**characterized**
**in that** for better adaptation to length changes of the groove as a result of bending the shaft the tube (5, 5b, 5a, 5w) has at least one free section in which the groove (4, 4') and the tube (5, 5b, 5a, 5w) are dimensioned relative to one another in such a way that the tube (5, 5b, 5a, 5w) has play in the axial direction, but also in respect of a rotation of the tube around its local longitudinal axis, by way of the tube having, in the straight extended state of the shaft (2), an exterior cross-section which is slightly smaller than an interior cross-section of the groove (4,4'), and
**in that** beyond and/or between the free section or free sections respectively are sections of the tube (5, 5b, 5a, 5w) which are inserted more firmly into the groove (4,4'), in that they are either pressed in exploiting the elasticity of the tube, or, alternatively or additionally, glued in with a not very strong adhesive.

2. Endoscope according to Claim 1, **characterized in that** the tube (5, 5b, 5a, 5w) in the free section has a higher deformability by means of
- being shaped in a bellows-like or spiral tube-like manner, and/or
- consisting of a material of higher elasticity, and/or
- a lower wall thickness.

3. Endoscope according to claim 2, **characterized in that** the tube (5, 5b, 5a, 5w) has two or more, in particular three or more, free sections with a higher deformability, which are connected by normal tube sections, whereby these normal sections, which are in particular all of equal length, are firmly retained in the groove (4, 4') by means of complementary sizing or an easily removable adhesive.

4. Endoscope according to claim 1 or 2, **characterized in that** the tube (5, 5b, 5a, 5w) is inserted into the groove (4, 4') having play therein over the entire length of the shaft (2), in particular the entire length of the groove (4, 4'), in particular has an exterior diameter, which is, at least in the straight extended state of the shaft (2), smaller than an interior diameter of the groove (4, 4') and during bending of the shaft (2) is secured
- against slipping out of the groove (4, 4') by means of two or more, in particular three or more, elastic retaining wings (49), and/or
- against sliding back into the groove at the distal end by a slip-back-preventer, in particular an annular disc (52), located at a distal end of the tube (5, 5b, 5a, 5w) and inserted into the distal end of the groove (4, 4').

5. Endoscope according to one of the claims 1 - 4, **characterized in that** the groove (4, 4')
- has rounded edges (45), and/or
- extends over the entire length of the shaft (2) from the distal end (21) to the proximal end (29) and, in particular, is continued preferably in an upper surface of the grip (3), and/or
- has a cross-section which is U- or Ω-shaped and constant along the shaft, and/or
- runs spirally around the shaft (2), wherein the spiral course covers an angle of between 0° and 180° preferably between 30° and 150°, and/or
- has a cross section which is U- and/or Ω -shaped and not constant along the shaft (2), in particular varying
• in shape between a U- and an Ω -shape, and/or
• in cross-sectional area, wherein in particular a locally minimal cross sectional area and/or a Ω-shape of the groove (4) is attained at or near the distal end (21), and/or
• at the proximal end (29) and/or in the grip (3) has an enlarged cross section for the purpose of enabling insertion of valves that are integrated into the tube (5) into valve holders (33b, 33a, 33w) of the grip (3).

6. Endoscope according to one of the claims 1 - 5, **characterized in that** the grip (3) has at least one valve holder (33b, 33a, 33w)
- for receiving a valve of a water channel (50w) or a valve of an air/suction channel (50a), in particular one or multiple recesses in the grip (3), whereby in particular the valves are integrated into valve blocks (53w, 53a) that are unitary with the tube (5, 5w, 5a) for facilitating their insertion into the valve holder (33b, 33a, 33w), and/or
- for fixing a proximal end (59b) of an instrument channel (50b) of the tube (5, 5b), wherein preferably the proximal end (59b) has a fixing structure (58, 58') for facilitating its insertion into one of t he valve holders.

7. Endoscope according to one of the claims 1 - 6, **characterized in that** the shaft (2)
- has a substantially symmetric cross-section, preferably a circular, elliptical or oval cross-section, and the groove (4, 4') lies in a plane of symmetry of the shaft (2),
- is manufactured from solid material, in particular a plastic, and/or
- has two grooves (4) suitable for receiving an instrument tube (5b).

8. Endoscope according to one of the Claims 1 - 7, **characterized in that** the groove (4, 4') contains, preferably along its entire extent, a center of area (CoA) of a convex hull (CH) or a completion (C) of a cross-section of the shaft (2).

9. Endoscope according to one of the Claims 1 - 8, **characterized in that** the tube (5, 205)
- has a cross-section which is complementary to a cross-section of the groove (4, 4'), and
- is made from an elastic material and has a diameter that is larger than a diameter of the groove (4) at least at two points along the shaft (2), one of these points lying at or near the distal end (21), the tube (5) thereby being fastened in the groove (4) due to elastic forces, and/or
- is fixed inside the groove (4, 4') by the not very strong adhesive.

## Revendications

1. Endoscope (1) pour l'examen mini-invasif ou le traitement chirurgical d'un patient, comprenant
- une tige tubulaire flexible (2) pour insertion dans le patient, avec une extrémité distale (21), et
- une poignée (3) de maintien de l'endoscope (1), une extrémité distale (31) de la poignée (3) étant fixée à une extrémité proximale (29) de la tige (2),
- dans lequel l'endoscope (1) est pourvu d'au moins un canal de travail (50b, 50w, 50a) pour guider intérieurement un instrument chirurgical et/ou transporter de l'eau ou de l'air à l'extrémité distale (21) de la tige ( 2) ou pour y fournir une aspiration,
- au moins une rainure (4, 4') s'étendant en essence axialement le long de la face extérieure de la tige (2) depuis le voisinage de l'extrémité proximale (29) jusqu'à l'extrémité distale (21), où la rainure (4, 4' ) se termine par un évidement (41, 41') dans l'extrémité distale (21), et
- le au moins un canal de travail (50b, 50w, 50a) est formé par au moins un tube creux (5, 5b, 5a, 5w) qui est logé de manière amovible dans la rainure (4, 4'), et
- dans lequel une enveloppe convexe (CH) ou une complétion (C) de la tige (2) à une forme géométrique simple sans le tube creux (5, 5b, 5a, 5w) est identique à une enveloppe convexe (CH) ou une finition (C) de l a tige avec le tube creux (5, 5b, 5a, 5w),
**caractérisé**
**en ce que** pour une meilleure adaptation aux changements de longueur de la rainure suite à la flexion de la tige, le tube (5, 5b, 5a, 5w) présente au moins une section libre dans laquelle la rainure (4, 4') et le tube (5 , 5b, 5a, 5w) sont dimensionnés l'un par rapport à l'autre de manière à ce que le tube (5, 5b, 5a, 5w) ait du jeu dans la direction axiale, mais aussi par rapport à une rotation du tube autour de son axe longitudinal local, au moyen du tube ayant, dans l'état étendu droit de la tige (2), une section transversale extérieure qui est légèrement plus petite qu'une section transversale intérieure de la rainure (4, 4'), et
**en ce qu'**au-delà et/ou entre la section libre ou les sections libres respectivement se trouvent des sections du tube (5, 5b, 5a, 5w) qui sont insérées plus fermement dans la rainure (4, 4'), en ce qu'elles sont enfoncées exploitant l'élasticité du tube, ou, alternativement ou en complément, collé avec un adhésif peu puissant.

2. Endoscope selon la revendication 1, **caractérisé en ce que** le tube (5, 5b, 5a, 5w) en la section libre présente une déformabilité supérieure grâce à
- ayant la forme d'un soufflet ou d'un tube en spirale, et/ou
- constitué d'un matériau d'élasticité plus élevée, et/ou
- une épaisseur de paroi inférieure.

3. Endoscope selon la revendication 2, **caractérisé en ce que** le tube (5, 5b, 5a, 5w) présente deux ou plus, en particulier trois ou plus, sections libres avec une déformabilité plus élevée, qui sont reliées par des sections de tube normales, ces sections des sections normales, notamment toutes de même longueur, sont solidement retenues dans la gorge (4, 4') au moyen d'un encollage complémentaire ou d'un adhésif facilement retirable.

4. Endoscope selon la revendication 1 ou 2, **caractérisé en ce que** le tube (5, 5b, 5a, 5w) est inséré dans la rainure (4, 4') en y jouant sur toute la longueur de la tige (2), en en particulier la longueur entière de la rainure (4, 4'), en particulier a un diamètre extérieur, qui est, au moins dans l'état étendu droit de la tige (2), plus petit qu'un diamètre intérieur de la rainure (4 , 4') et pendant la flexion de la tige (2) est fixé
- contre le glissement hors de la rainure (4, 4') au moyen de deux ou plusieurs, en particulier trois ou plus, ailes de retenue élastiques (49), et/ou
- contre le retour dans la rainure à l'extrémité distale par un dispositif anti-retour, en particulier un disque annulaire (52), situé à une extrémité distale du tube (5, 5b, 5a, 5w) et inséré dans l'extrémité distale de la rainure (4, 4').

5. Endoscope selon l'une des revendications 1 à 4, **caractérisé en ce que** la rainure (4, 4')
- a des bords arrondis (45), et/ou
- s'étend sur toute la longueur de la tige (2) de l'extrémité distale (21) à l'extrémité proximale (29) et, en particulier, se poursuit de préférence dans une surface supérieure de la poignée (3), et/ou
- a une section transversale en forme de U ou de Ω et constante le long de la tige, et/ou
- s'étend en spirale autour de la tige (2), la course en spirale couvrant un angle compris entre 0° et 180°, de préférence entre 30° et 150°, et/ou
- a une section transversale en forme de U et/ou de Ω et non constante le long de la tige (2), notamment variable
• en forme de U et de Ω, et/ou
• dans une zone de section transversale, dans laquelle en particulier une zone de section transversale localement minimale et/ou une forme en Ω de la rainure (4) est atteinte au niveau ou à proximité de l'extrémité distale (21), et/ou
• présente à l'extrémité proximale (29) et/ou dans la poignée (3) une section élargie dans le but de permettre l'insertion de valves intégrées dans le tube (5) dans des porte-valves (33b, 33a, 33w ) de la poignée (3).

6. Endoscope selon l'une des revendications 1 à 5, **caractérisé en ce que** la poignée (3) comporte au moins un porte-valve (33b, 33a, 33w)
- pour recevoir une valve d'un canal d'eau (50w) ou une valve d'un canal d'air/d'aspiration (50a), en particulier un ou plusieurs évidements dans la poignée (3), les valves étant notamment intégrées dans des blocs de valve (53w, 53a) solidaires du tube (5, 5w, 5a) pour faciliter leur insertion dans le porte-valve (33b, 33a, 33w), et/ou
- pour fixer une extrémité proximale (59b) d'un canal d'instrument (50b) du tube (5, 5b), dans lequel de préférence l'extrémité proximale (59b) a une structure de fixation (58, 58') pour faciliter son insertion dans l'un des porte-valves.

7. Endoscope selon l'une des revendications 1 à 6, **caractérisé en ce que** la tige (2)
- présente une section essentiellement symétrique, de préférence une section circulaire, elliptique ou ovale, et la rainure (4, 4') est située dans un plan de symétrie de la tige (2),
- est fabriqué à partir d'un matériau solide, notamment un plastique, et/ou
- présente deux rainures (4) aptes à recevoir un tube instrument (5b).

8. Endoscope selon l'une des revendications 1 à 7, **caractérisé en ce que** la rainure (4, 4') contient, de préférence sur toute son étendue, un centre d'aire (CoA) d'une enveloppe convexe (CH) ou une complétion (C) d'une section transversale de la tige (2).

9. Endoscope selon l'une des revendications 1 à 8, **caractérisé en ce que** le tube (5, 205)
- présente une section transversale complémentaire d'une section transversale de la rainure (4, 4'), et
- est réalisé en un matériau élastique et présente un diamètre supérieur à un diamètre de la reinure (4) en au moins deux points le long de la tige (2), l'un de ces points se trouvant au niveau ou à proximité de l'extrémité distale (21), le tube (5) étant ainsi fixé dans la rainure (4) sous l'effet de forces élastiques, et/ou
- est fixé à l'intérieur de la rainure (4, 4') par l'adhésif peu résistant.
